# EUROPEAN PATENT APPLICATION

(11) **EP 4 670 742 A1**
(43) Date of publication of application: **31.12.2025**
(21) Application number: 25185149.9
(22) Date of filing: 25.06.2025
(51) Int. Cl.: A61K 47/68, A61P 35/00, C07K 16/28

(54) **ANTI-CD79B ANTIBODY DRUG CONJUGATES**

(30) Priority: 25.06.2024 US 202463663844 P
(71) Applicant: AbbVie Inc., North Chicago, IL 60064 (US)
(72) Inventor: Belair, David Gregory, Meadows, IL 60008 (US); Izeradjene, Kamel, Gurnee, IL 60031 (US); Ji, Cheng, Wilmette, IL 60091 (US); McCluskey, Andrew Jeffrey, Holden, MA 01520 (US); Reilly, Regina M., Libertyville, IL 60048 (US); Ren, Siyuan, Hills, IL, 60061 (US); Touw, Debra S., Worcester, MA 01606 (US); Wolke, Malerie, Gurnee, IL 60031 (US); Xiao, Yu, Wadsworth, IL 60083 (US)
(74) Representative: Hoffmann Eitle

(57) **Abstract**

The present disclosure relates to anti-CD79b antibodies and anti-CD79b antibody drug conjugates (anti-CD79b ADCs) comprising thereof, where the anti-CD79b ADCs further comprise a topoisomerase 1 inhibitor (Topli) drug as a payload. The present disclosure further relates to methods of using such anti-CD79b ADCs for treating B-cell non-Hodgkin lymphoma (B-NHL), diffuse large B cell lymphoma (DLBCL), Burkitt's lymphoma, mantle cell lymphoma, and follicular lymphoma.

## Description

### CROSS REFERENCE TO RELATED APPLICATIONS

This application claims priority to and benefit of United States Provisional Application No. 63/663,844 filed on June 25, 2024, the content of which is hereby incorporated by reference in its entirety.

### SEQUENCE LISTING

The instant application contains a Sequence Listing which has been submitted electronically in XML format and is hereby incorporated by reference in its entirety. Said XML copy, created on June 24, 2025, is named ABV21655WOO1_ST26.xml and is 158,263 bytes in size.

### TECHNICAL FIELD

The present disclosure relates to anti-CD79b antibodies and anti-CD79b Top1i antibody drug conjugates (anti-CD79b Top1i ADCs) comprising the anti-CD79b antibodies and a topoisomerase 1 inhibitor (Top1i) drug.

### BACKGROUND

CD79b is overexpressed in most newly diagnosed B-cell non-Hodgkin lymphomas (B-NHL). CD79 is a heterodimeric protein that comprises two transmembrane subunits, CD79a and CD79b. The B-cell receptor (BCR) transduces signals intracellularly through CD79a-CD79b heterodimer and signals originating from BCR, which can sustain malignant B-cell growth and survival. CD79b is part of the signaling component of the BCR and is an attractive antibody drug conjugate (ADC) target because it is expressed on nearly all major subtypes of B-NHL and is rapidly trafficked to lysosomes through major histocompatibility complex (MCH) Class II antigen presentation.

ADCs represent a class of therapeutics comprising an antibody conjugated to a cytotoxic drug, often referred to as a "payload," via a chemical linker. The therapeutic concept of ADCs is to combine the binding capability of an antibody with a cytotoxic drug for targeted delivery of the cytotoxic drug to tumor cells. This is achieved through the binding of the antibody part of the ADCs to a target surface antigen, including target surface antigens that are overexpressed or amplified in the tumor cells.

There remains a need in the art for developing potent anti-CD79b Top1i ADCs that provide targeted delivery in treating B-cell malignancies.

### SUMMARY

The present disclosure provides anti-CD79b antibodies and anti-CD79b Top1i ADCs comprising anti-CD79b antibodies. The anti-CD79b Top1i ADCs further comprise a Top1i drug as a payload.

As will be further described subsequently in the present disclosure, embodiments of the presently disclosed anti-CD79b Top1i ADC have potent anti-proliferative and cytotoxic activities against various B-cell malignant cell lines.

In a first aspect, the present disclosure provides an anti-CD79b Top1i ADC of the following structure: 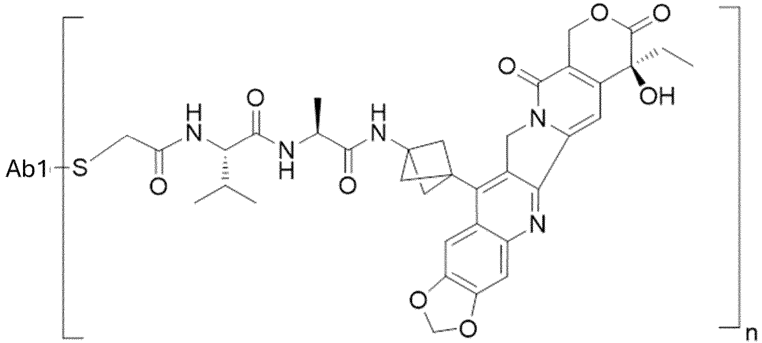 wherein n is an integer from 1 to 8
wherein Ab1 is an anti-CD79b antibody comprising a heavy chain variable region comprising a VH-CDR1, a VH-CDR2, and a VH-CDR3; and a light chain variable region comprising a VL-CDR1, a VL-CDR2, and a VL-CDR3; and wherein:
VH-CDR1 has the amino acid sequence of SEQ ID NO: 1;
VH-CDR2 has the amino acid sequence of SEQ ID NO: 2;
VH-CDR3 has the amino acid sequence of SEQ ID NO: 3;
VL-CDR1 has the amino acid sequence of SEQ ID NO: 4;
VL-CDR2 has the amino acid sequence of SEQ ID NO: 5; and
VL-CDR3 has the amino acid sequence of SEQ ID NO: 6.

A second aspect comprises the anti-CD79b ADC of the first aspect, wherein Ab1 is an IgG₁ isotype and comprises: a heavy chain variable region having the amino acid sequence of SEQ ID NO: 7; and a light chain variable region having the amino acid sequence of SEQ ID NO: 8.

A third aspect comprises the anti-CD79b ADC of the first aspect, wherein Ab1 is an IgG₁ isotype and comprises: a heavy chain having the amino acid sequence of SEQ ID NO: 9 or SEQ ID NO: 13; and a light chain having the amino acid sequence of SEQ ID NO: 10.

A fourth aspect comprises the anti-CD79b ADC of the third aspect, wherein Ab1 comprises: a heavy chain having the amino acid sequence of SEQ ID NO: 13; and a light chain having the amino acid sequence of SEQ ID NO: 10.

A fifth aspect comprises the anti-CD79b ADC of the fourth aspect, wherein n is 6.

A sixth aspect comprises the anti-CD79b ADC of the third aspect, wherein Ab1 comprises: a heavy chain having the amino acid sequence of SEQ ID NO: 9; and a light chain having the amino acid sequence of SEQ ID NO: 10.

A seventh aspect comprises the anti-CD79b ADC of the sixth aspect, wherein n is 6.

An eighth aspect comprises the anti-CD79b ADC of the first or second aspect, wherein the anti-CD79b antibody comprises two identical heavy chains and two identical light chains, wherein each heavy chain comprises the amino acid sequence of SEQ ID NO: 9 or SEQ ID NO: 13; and wherein each light chain comprises the amino acid sequence of SEQ ID NO: 10

A nineth aspect comprises the anti-CD79b ADC of the first or second aspect, wherein the anti-CD79b antibody comprises a human kappa light chain constant region.

A tenth aspect comprises a pharmaceutical composition comprising the anti-CD79b ADC of any one of the first through nineth aspects and a pharmaceutically acceptable carrier.

An eleventh aspect comprises a method of treating a disease, the method comprising administering an anti-CD79b ADC of any one of the first through nineth aspects to a patient in need thereof.

A twelfth aspect comprises an antibody that specifically binds to human CD79b, wherein the antibody comprises: a heavy chain variable region comprising the amino acid sequence of SEQ ID NO:7; and a light chain variable region comprising the amino acid sequence of SEQ ID NO:8.

A thirteenth aspect comprises the antibody of the twelfth aspect, wherein the antibody is an IgG₁ isotype, and wherein the VH-CDR1 comprises the amino acid sequence of SEQ ID NO: 1, the VH-CDR2 comprises the amino acid sequence of SEQ ID NO:2, the VH-CDR3 comprises the amino acid sequence of SEQ ID NO:3, the VL-CDR1 comprises the amino acid sequence of SEQ ID NO:4, the VL-CDR2 comprises the amino acid sequence of SEQ ID NO:5, and the VL-CDR3 comprises the amino acid sequence of SEQ ID NO:6.

A fourteenth aspect comprises the antibody of the twelfth or thirteenth aspect, wherein the antibody comprises a human kappa light chain constant region.

A fifteenth aspect comprises the antibody of any one of the twelfth through fourteenth aspects, wherein the antibody comprises a heavy chain variable region comprising the amino acid sequence of SEQ ID NO:7 and a light chain variable region comprising the amino acid sequence of SEQ ID NO:8.

A sixteenth aspect comprises the antibody of any one of the twelfth through fifteenth aspects, wherein the antibody comprises a heavy chain comprising the amino acid sequence of SEQ ID NO: 9 or SEQ ID NO: 13 and a light chain comprising the amino acid sequence of SEQ ID NO:10.

A seventeenth aspect comprises the antibody of the sixteenth aspect, wherein the antibody comprises a heavy chain comprising the amino acid sequence of SEQ ID NO: 13 and a light chain comprising the amino acid sequence of SEQ ID NO: 10.

An eighteenth aspect comprises the antibody of the sixteenth aspect, wherein the antibody comprises a heavy chain comprising the amino acid sequence of SEQ ID NO: 9 and a light chain comprising the amino acid sequence of SEQ ID NO: 10.

A nineteenth aspect comprises a polynucleotide or a set of polynucleotides encoding the antibody of any one of the twelfth through eighteenth aspects or a portion thereof.

A twentieth aspect comprises a vector or a set of vectors comprising the polynucleotide or set of polynucleotides of the nineteenth aspect.

A twenty-first aspect comprises a host cell comprising the polynucleotide or set of polynucleotides of the nineteenth aspect or the vector or set of vectors of the twentieth aspect.

A twenty-second aspect comprises a pharmaceutical composition comprising the antibody of any one of the twelfth through eighteenth aspects and a pharmaceutically acceptable carrier.

A twenty-third aspect comprises a process for producing an anti-CD79b antibody-drug conjugate, comprising conjugating an anti-CD79b antibody of any one of the twelfth to eighteenth aspects to a Topli payload shown as Formula 2 via a linker shown as Formula 3.

A twenty-fourth aspect comprises a composition comprising an anti-CD79b antibody-drug conjugate wherein the DAR is about 4.

### BRIEF DESCRIPTION OF THE DRAWINGS

**FIG.** 1 depicts the results of an *in vivo* assay performed on ADC-1.
**FIG.** 2 depicts the results of an *in vivo* assay performed on ADC-1 compared to Polatuzumab vedotin-piiq.

### DETAILED DESCRIPTION

Disclosed herein are embodiments including anti-CD79b antibodies and anti-CD79b Top1i ADCs comprising anti-CD79b antibodies and a Top1i drug. Embodiments also include Top1i linker-drugs useful for synthesizing the anti-CD79b Top1i ADCs, methods of making the anti-CD79b Top1i ADCs, and methods of using the anti-CD79b Top1i ADCs.

### 1.1 Anti-CD79b Antibody

Embodiments of the present disclosure are directed to an anti-CD79b antibody, Ab1, that specifically binds CD79b. In certain embodiments, the anti-CD79b antibody specifically binds human CD79b. In embodiments, the anti-CD79b antibody does not specifically bind human CD79a. In embodiments, the anti-CD79b antibody does not have cross-reactivity to mouse, rat, rabbit, dog, or cynomolgus monkey CD79b.

In embodiments, the anti-CD79b antibody comprises a heavy chain variable region complementarity determining region 1 (VH-CDR1), a heavy chain variable region complementarity determining region 2 (VH-CDR2), and a heavy chain variable region complementarity determining region 3 (VH-CDR3) as set forth in a heavy chain variable region (VH) comprising the amino acid sequence of SEQ ID NO:7; and a light chain variable region complementarity determining region 1 (VL-CDR1), a light chain variable region complementarity determining region 2 (VL-CDR2), and a light chain variable region complementarity determining region 3 (VL-CDR3) as set forth in a light chain variable region (VL) comprising the amino acid sequence of SEQ ID NO:8.

In certain embodiments, the anti-CD79b antibody comprises a VH-CDR1 comprising the amino acid sequence of SEQ ID NO: 1, a VH-CDR2 comprising the amino acid sequence of SEQ ID NO:2, a VH-CDR3 comprising the amino acid sequence of SEQ ID NO:3, a VL-CDR1 comprising the amino acid sequence of SEQ ID NO:4, a VL-CDR2 comprising the amino acid sequence of SEQ ID NO:5, and a VL-CDR3 comprising the amino acid sequence of SEQ ID NO:6. In certain embodiments, the anti-CD79b antibody comprises a VH-CDR1 comprising the amino acid sequence of SEQ ID NO: 14, a VH-CDR2 comprising the amino acid sequence of SEQ ID NO: 15, a VH-CDR3 comprising the amino acid sequence of SEQ ID NO: 16, a VL-CDR1 comprising the amino acid sequence of SEQ ID NO: 17, a VL-CDR2 comprising the amino acid sequence of SEQ ID NO: 18, and a VL-CDR3 comprising the amino acid sequence of SEQ ID NO: 19.

In certain embodiments, the anti-CD79b antibody comprises a VH comprising the amino acid sequence of SEQ ID NO:7 and/or a VL comprising the amino acid sequence of SEQ ID NO:8. In certain embodiments, the anti-CD79b antibody comprises a VH comprising the amino acid sequence of SEQ ID NO:7 and a VL comprising the amino acid sequence of SEQ ID NO:8.

In certain embodiments, the anti-CD79b antibody comprises a heavy chain comprising the amino acid sequence of SEQ ID NO: 9 or SEQ ID NO: 13 and a light chain comprising the amino acid sequence of SEQ ID NO: 10. In embodiments, the predominant species of the heavy chain of Ab1 comprises a heavy chain sequence which lacks a C-terminal lysine that comprises a heavy chain sequence comprising the amino acid sequence of SEQ ID NO: 13.

In certain embodiments, the anti-CD79b antibody disclosed herein is a monoclonal antibody. In certain embodiments, the anti-CD79b antibody is an IgG₁ antibody. In certain embodiments, the anti-CD79b antibody comprises a kappa light chain constant region (*e.g.,* a human kappa light chain constant region). In certain embodiments, the anti-CD79b antibody was derived from a fully recombinant humanized monoclonal IgG₁ antibody produced in Alivamab^{®} (Ablexis) mice and comprising a human kappa light chain constant region.

In certain embodiments, the anti-CD79b antibody is designated as "Ab1" herein and comprises the amino acid sequences and Kabat-designated CDRs and IMGT-designated CDRs set forth in **Table 1.**

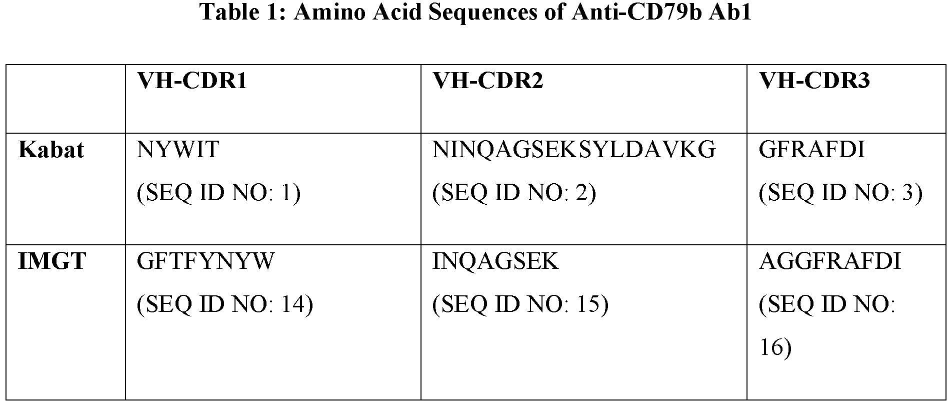

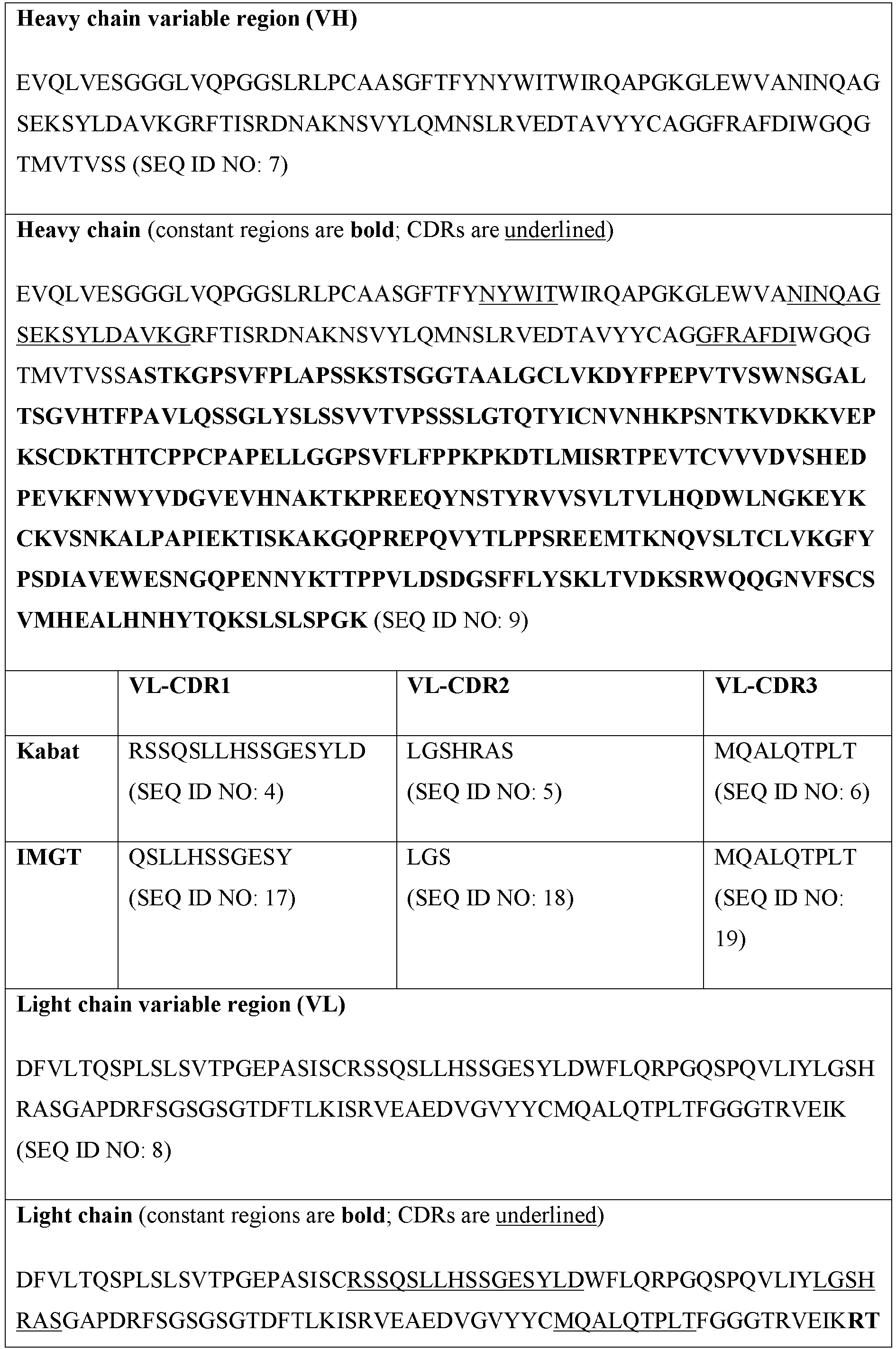

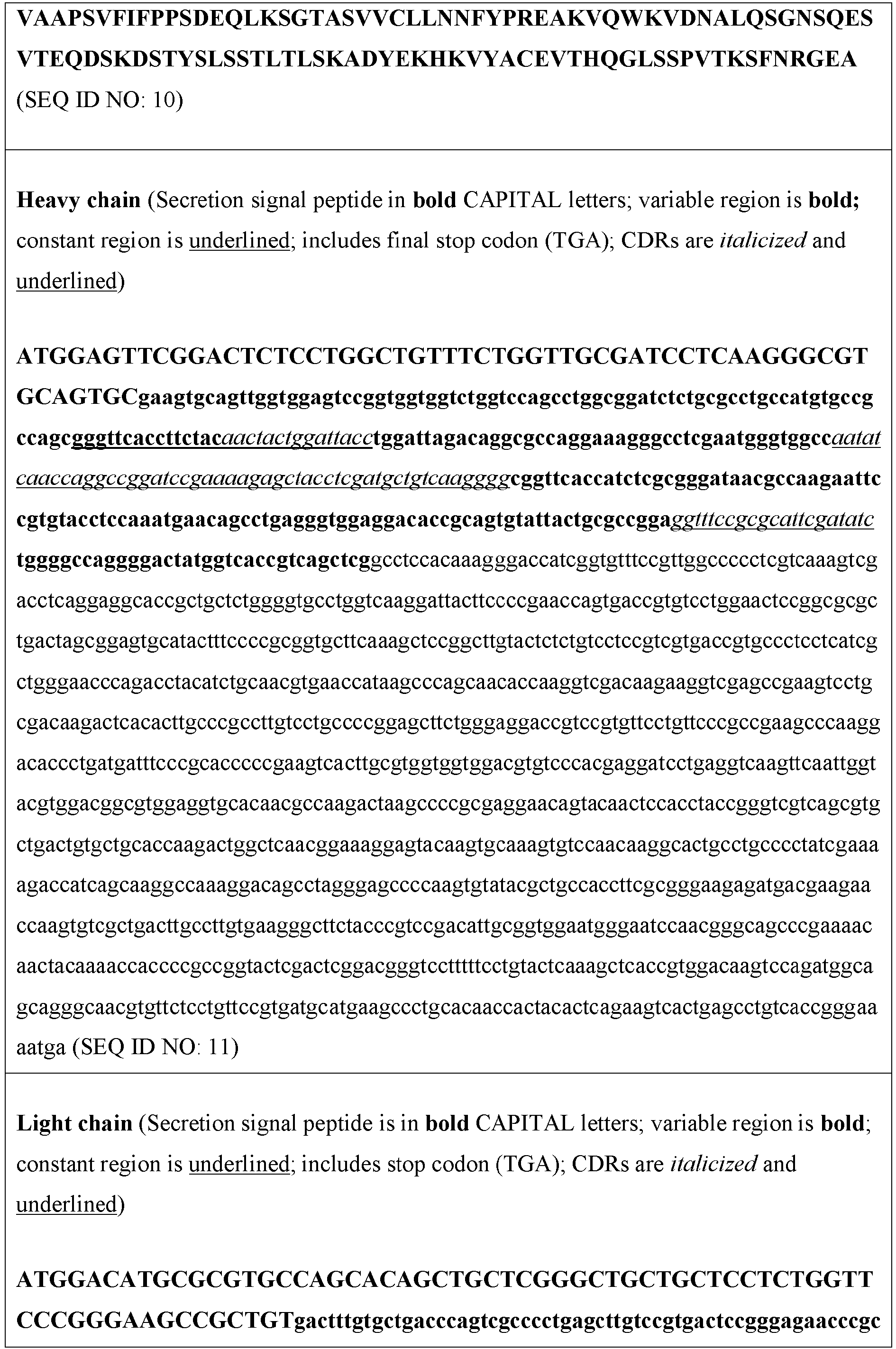

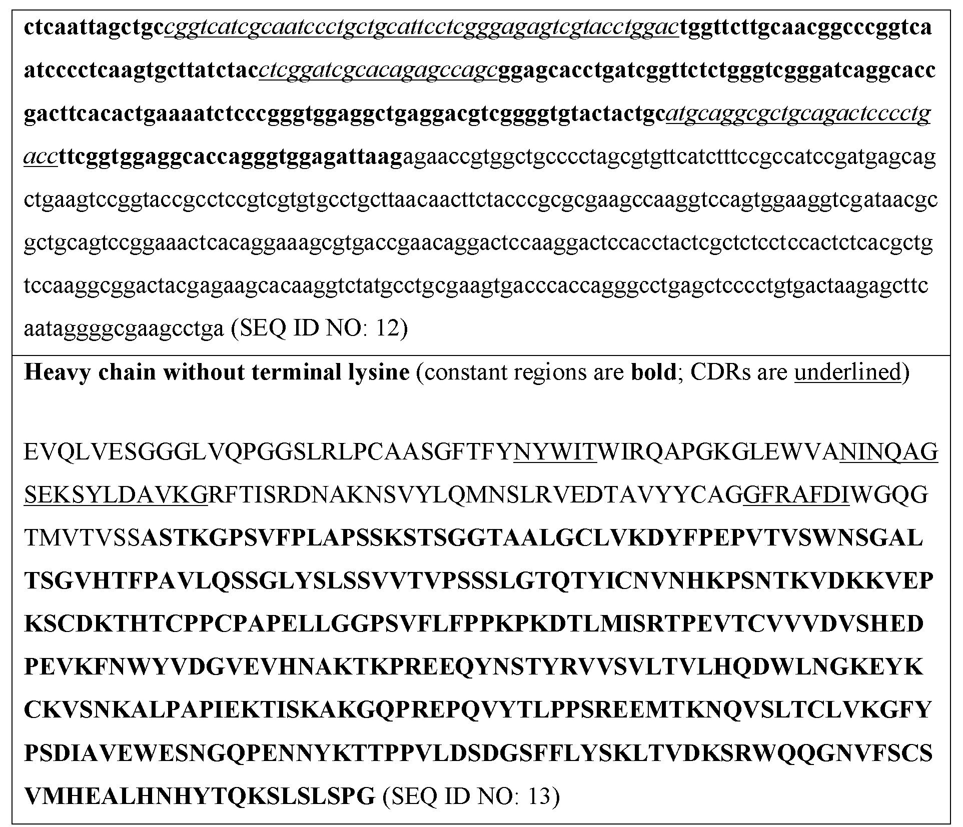

In embodiments, the anti-CD79b antibody may be in a format, such as an IgG1 or IgG4 format, that has been modified to confer desired properties, such as having the Fc mutated to reduce or "silence" effector function or extend half-life. In embodiments where half-life extending and/or Fc silencing mutations are introduced, the anti-CD79b antibody may comprise the following heavy chain sequences in the antibody combinations shown in **Table 2:**

**Table 2: Fc Mutated Sequences of Anti-CD79b Ab1**

| | |
|---|---|
| **Ab1 heavy chain IgG1 STR** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDTLMISRTPEVTCVVVDVSHEDPEVKFNW YVDGVEVHNAKTKPREEQYNSTYRVVSVLTVLHQDWLNGKEYKC |
| | KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHNHYTQKSLSLSPGK (SEQ ID NO: 20) |
| **Ab1 heavy chain IgG1 STR without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHNHYTQKSLSLSPG (SEQ ID NO: 21) |
| **Ab1 heavy chain IgG1 LALA** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APEAAGGPSVFLFPPKPKDTLMISRTPEVTCVVVDVSHEDPEVKFN WYVDGVEVHNAKTKPREEQYNSTYRVVSVLTVLHQDWLNGKEYK CKVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVMHEALHNHYTQKSLSLSPGK (SEQ ID NO: 22) |
| **Ab1 heavy chain IgG1 LALA without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP |
| | APEAAGGPSVFLFPPKPKDTLMISRTPEVTCVVVDVSHEDPEVKFN WYVDGVEVHNAKTKPREEQYNSTYRVVSVLTVLHQDWLNGKEYK CKVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVMHEALHNHYTQKSLSLSPG (SEQ ID NO: 23) |
| **Ab1 heavy chain IgG1 YTE** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDTLYITREPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHNHYTQKSLSLSPGK (SEQ ID NO: 24) |
| **Ab1 heavy chain IgG1 YTE without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDTLYITREPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHNHYTQKSLSLSPG (SEQ ID NO: 25) |
| **Ab1 heavy chain IgG1 STR and YTE** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG |
| | TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDTLYITREPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHNHYTQKSLSLSPGK (SEQ ID NO: 26) |
| **Ab1 heavy chain IgG1 STR and YTE without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDTLYITREPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHNHYTQKSLSLSPG (SEQ ID NO: 27) |
| **Ab1 heavy chain IgG1 LALA and YTE** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APEAAGGPSVFLFPPKPKDTLYITREPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHNHYTQKSLSLSPGK (SEQ ID NO: 28) |
| **Ab1 heavy chain IgG1 LALA and YTE without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APEAAGGPSVFLFPPKPKDTLYITREPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHNHYTQKSLSLSPG (SEQ ID NO: 29) |
| **Ab1 heavy chain IgG1 REW** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDTLMISRTPEVTCVVVDVSHEDPEVKFNW YVDGVEVHNAKTKPREEQYNSTYRVVSVLTVLHRDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVEHEALHWHYTQKSLSLSPGK (SEQ ID NO: 30) |
| **Ab1 heavy chain IgG1 REW without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDTLMISRTPEVTCVVVDVSHEDPEVKFNW YVDGVEVHNAKTKPREEQYNSTYRVVSVLTVLHRDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVEHEALHWHYTQKSLSLSPG |
| | (SEQ ID NO: 31) |
| **Ab1 heavy chain IgG1 STR and REW** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDTLMISRTPEVTCVVVDVSHEDPEVKFNW YVDGVEVHNAKTKPREEQYNSTYRVVSVLTVLHRDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVEHEALHWHYTQKSLSLSPGK (SEQ ID NO: 32) |
| **Ab1 heavy chain IgG1 STR and REW without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDTLMISRTPEVTCVVVDVSHEDPEVKFNW YVDGVEVHNAKTKPREEQYNSTYRVVSVLTVLHRDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVEHEALHWHYTQKSLSLSPG (SEQ ID NO: 33) |
| **Ab1 heavy chain IgG1 LALA and REW** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APEAAGGPSVFLFPPKPKDTLMISRTPEVTCVVVDVSHEDPEVKFN WYVDGVEVHNAKTKPREEQYNSTYRVVSVLTVLHRDWLNGKEYK CKVSNKALPAPIEKTISKAKGQPREPQVYTLPPSREEMTKNQVSLTC |
| | LVKGFYPSDIAVEWESNGQPENNYKTTPPVLDSDGSFFLYSKLTVD KSRWQQGNVFSCSVEHEALHWHYTQKSLSLSPGK (SEQ ID NO: 34) |
| **Ab1 heavy chain IgG1 LALA and REW without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APEAAGGPSVFLFPPKPKDTLMISRTPEVTCVVVDVSHEDPEVKFN WYVDGVEVHNAKTKPREEQYNSTYRVVSVLTVLHRDWLNGKEYK CKVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVEHEALHWHYTQKSLSLSPG (SEQ ID NO: 35) |
| **Ab1 heavy chain IgG1 LS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVLHEALHSHYTQKSLSLSPGK (SEQ ID NO: 36) |
| **Ab1 heavy chain IgG1 LS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDTLMISRTPEVTCVVVDVSHEDPEVKFNW |
| | YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVLHEALHSHYTQKSLSLSPG (SEQ ID NO: 37) |
| **Ab1 heavy chain IgG1 STR and LS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVLHEALHSHYTQKSLSLSPGK (SEQ ID NO: 38) |
| **Ab1 heavy chain IgG1 STR and LS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDTLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVLHEALHSHYTQKSLSLSPG (SEQ ID NO: 39) |
| **Ab1 heavy chain IgG1 LALA and LS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS |
| | VVTVPSSSLGTQTYICNVNHKPSNTKVDKKVEPKSCDKTHTCPPCP APEAAGGPSVFLFPPKPKDTLMISRTPEVTCVVVDVSHEDPEVKFN WYVDGVEVHNAKTKPREEQYNSTYRVVSVLTVLHQDWLNGKEYK CKVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVLHEALHSHYTQKSLSLSPGK (SEQ ID NO: 40) |
| **Ab1 heavy chain IgG1 LALA and LS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APEAAGGPSVFLFPPKPKDTLMISRTPEVTCVVVDVSHEDPEVKFN WYVDGVEVHNAKTKPREEQYNSTYRVVSVLTVLHQDWLNGKEYK CKVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVLHEALHSHYTQKSLSLSPG (SEQ ID NO: 41) |
| **Ab1 heavy chain IgG1 QL** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDQLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVLHEALHNHYTQKSLSLSPGK (SEQ ID NO: 42) |
| **Ab1 heavy chain IgG1 QL** | EVQLVESGGGLVQPGGSLRLPCAASGFTFYNYWITWIRQAPGKGLE WVANINQAGSEKSYLDAVKGRFTISRDNAKNSVYLQMNSLRVEDT |
| **without terminal lysine** | AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDQLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVLHEALHNHYTQKSLSLSPG (SEQ ID NO: 43) |
| **Ab1 heavy chain IgG1 STR and QL** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDQLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVLHEALHNHYTQKSLSLSPGK (SEQ ID NO: 44) |
| **Ab1 heavy chain IgG1 STR and QL without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDQLMISRTPEVTCVVVDVSHEDPEVKFNW YVDGVEVHNAKTKPREEQYNSTYRVVSVLTVLHQDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVLHEALHNHYTQKSLSLSPG (SEQ ID NO: 45) |
| **Ab1 heavy chain IgG1 LALA and QL** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APEAAGGPSVFLFPPKPKDQLMISRTPEVTCVVVDVSHEDPEVKFN WYVDGVEVHNAKTKPREEQYNSTYRVVSVLTVLHQDWLNGKEYK CKVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVLHEALHNHYTQKSLSLSPGK (SEQ ID NO: 46) |
| **Ab1 heavy chain IgG1 LALA and QL without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APEAAGGPSVFLFPPKPKDQLMISRTPEVTCVVVDVSHEDPEVKFN WYVDGVEVHNAKTKPREEQYNSTYRVVSVLTVLHQDWLNGKEYK CKVSNKALPAPIEKTISKAKGQPREPQVYTLPPSREEMTKNQVSLTC LVKGFYPSDIAVEWESNGQPENNYKTTPPVLDSDGSFFLYSKLTVD KSRWQQGNVFSCSVLHEALHNHYTQKSLSLSPG (SEQ ID NO: 47) |
| **Ab1 heavy chain IgG1 DHS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDTLMISRTPEVTCVVVDVSHEDPEVKFNW YVDGVEVHNAKTKPREEQYNSTYRVVSVLTVDHHDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHSHYTQKSLSLSPGK |
| | (SEQ ID NO: 48) |
| **Ab1 heavy chain IgG1 DHS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APELLGGPSVFLFPPKPKDTLMISRTPEVTCVVVDVSHEDPEVKFNW YVDGVEVHNAKTKPREEQYNSTYRVVSVLTVDHHDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHSHYTQKSLSLSPG (SEQ ID NO: 49) |
| **Ab1 heavy chain IgG1 STR and DHS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDTLMISRTPEVTCVVVDVSHEDPEVKFNW YVDGVEVHNAKTKPREEQYNSTYRVVSVLTVDHHDWLNGKEYKC KVSNKALPAPIEKTISKAKGQPREPQVYTLPPSREEMTKNQVSLTCL VKGFYPSDIAVEWESNGQPENNYKTTPPVLDSDGSFFLYSKLTVDK SRWQQGNVFSCSVMHEALHSHYTQKSLSLSPGK (SEQ ID NO: 50) |
| **Ab1 heavy chain IgG1 STR and DHS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APESTRGPSVFLFPPKPKDTLMISRTPEVTCVVVDVSHEDPEVKFNW YVDGVEVHNAKTKPREEQYNSTYRVVSVLTVDHHDWLNGKEYKC KVSNKALPAPIEKTISKAKGQPREPQVYTLPPSREEMTKNQVSLTCL |
| | VKGFYPSDIAVEWESNGQPENNYKTTPPVLDSDGSFFLYSKLTVDK SRWQQGNVFSCSVMHEALHSHYTQKSLSLSPG (SEQ ID NO: 51) |
| **Ab1 heavy chain IgG1 LALA and DHS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APEAAGGPSVFLFPPKPKDTLMISRTPEVTCVVVDVSHEDPEVKFN WYVDGVEVHNAKTKPREEQYNSTYRVVSVLTVDHHDWLNGKEY KCKVSNKALPAPIEKTISKAKGQPREPQVYTLPPSREEMTKNQVSLT CLVKGFYPSDIAVEWESNGQPENNYKTTPPVLDSDGSFFLYSKLTV DKSRWQQGNVFSCSVMHEALHSHYTQKSLSLSPGK (SEQ ID NO: 52) |
| **Ab1 heavy chain IgG1 LALA and DHS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPSSKSTSGG TAALGCLVKDYFPEPVTVSWNSGALTSGVHTFPAVLQSSGLYSLSS VVTVPSSSLGTQTYICNVNHKPSNTKVDKKVEPKSCDKTHTCPPCP APEAAGGPSVFLFPPKPKDTLMISRTPEVTCVVVDVSHEDPEVKFN WYVDGVEVHNAKTKPREEQYNSTYRVVSVLTVDHHDWLNGKEY KCKVSNKALPAPIEKTISKAKGQPREPQVYTLPPSREEMTKNQVSLT CLVKGFYPSDIAVEWESNGQPENNYKTTPPVLDSDGSFFLYSKLTV DKSRWQQGNVFSCSVMHEALHSHYTQKSLSLSPG (SEQ ID NO: 53) |
| **Ab1 heavy chain IgG4 STR** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDTLMISRTPEVTCVVVDVSQEDPEVQFNWYVD |
| | GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLGK (SEQ ID NO: 54) |
| **Ab1 heavy chain IgG4 STR without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLG (SEQ ID NO: 55) |
| **Ab1 heavy chain IgG4 FALA** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPE AAGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLGK (SEQ ID NO: 56) |
| **Ab1 heavy chain IgG4 FALA without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS |
| | VVTVPSSSLGTKTYTCNVDHKPSNTKVDKRVESKYGPPCPSCPAPE AAGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLG (SEQ ID NO: 57) |
| **Ab1 heavy chain IgG4 YTE** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDTLYITREPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLGK (SEQ ID NO: 58) |
| **Ab1 heavy chain IgG4 YTE without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDTLYITREPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLG (SEQ ID NO: 59) |
| **Ab1 heavy chain IgG4 STR and YTE** | EVQLVESGGGLVQPGGSLRLPCAASGFTFYNYWITWIRQAPGKGLE WVANINQAGSEKSYLDAVKGRFTISRDNAKNSVYLQMNSLRVEDT |
| | AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDTLYITREPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLGK (SEQ ID NO: 60) |
| **Ab1 heavy chain IgG4 STR and YTE without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDTLYITREPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLG (SEQ ID NO: 61) |
| **Ab1 heavy chain IgG4 FALA and YTE** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPE AAGGPSVFLFPPKPKDTLYITREPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLGK (SEQ ID NO: 62) |
| **Ab1 heavy chain IgG4 FALA and YTE without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPE AAGGPSVFLFPPKPKDTLYITREPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLG (SEQ ID NO: 63) |
| **Ab1 heavy chain IgG4 REW** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDTLMISRTPEVTCVVVDVSQEDPEVQFNWYVD GVEVHNAKTKPREEQFNSTYRVVSVLTVLHRDWLNGKEYKCKVSN KGLPSSIEKTISKAKGQPREPQVYTLPPSQEEMTKNQVSLTCLVKGF YPSDIAVEWESNGQPENNYKTTPPVLDSDGSFFLYSRLTVDKSRWQ EGNVFSCSVEHEALHWHYTQKSLSLSLGK (SEQ ID NO: 64) |
| **Ab1 heavy chain IgG4 REW without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDTLMISRTPEVTCVVVDVSQEDPEVQFNWYVD GVEVHNAKTKPREEQFNSTYRVVSVLTVLHRDWLNGKEYKCKVSN KGLPSSIEKTISKAKGQPREPQVYTLPPSQEEMTKNQVSLTCLVKGF YPSDIAVEWESNGQPENNYKTTPPVLDSDGSFFLYSRLTVDKSRWQ EGNVFSCSVEHEALHWHYTQKSLSLSLG |
| | (SEQ ID NO: 65) |
| **Ab1 heavy chain IgG4 STR and REW** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDTLMISRTPEVTCVVVDVSQEDPEVQFNWYVD GVEVHNAKTKPREEQFNSTYRVVSVLTVLHRDWLNGKEYKCKVSN KGLPSSIEKTISKAKGQPREPQVYTLPPSQEEMTKNQVSLTCLVKGF YPSDIAVEWESNGQPENNYKTTPPVLDSDGSFFLYSRLTVDKSRWQ EGNVFSCSVEHEALHWHYTQKSLSLSLGK (SEQ ID NO: 66) |
| **Ab1 heavy chain IgG4 STR and REW without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDTLMISRTPEVTCVVVDVSQEDPEVQFNWYVD GVEVHNAKTKPREEQFNSTYRVVSVLTVLHRDWLNGKEYKCKVSN KGLPSSIEKTISKAKGQPREPQVYTLPPSQEEMTKNQVSLTCLVKGF YPSDIAVEWESNGQPENNYKTTPPVLDSDGSFFLYSRLTVDKSRWQ EGNVFSCSVEHEALHWHYTQKSLSLSLG (SEQ ID NO: 67) |
| **Ab1 heavy chain IgG4 FALA and REW** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPE AAGGPSVFLFPPKPKDTLMISRTPEVTCVVVDVSQEDPEVQFNWYV DGVEVHNAKTKPREEQFNSTYRVVSVLTVLHRDWLNGKEYKCKVS NKGLPSSIEKTISKAKGQPREPQVYTLPPSQEEMTKNQVSLTCLVKG |
| | FYPSDIAVEWESNGQPENNYKTTPPVLDSDGSFFLYSRLTVDKSRW QEGNVFSCSVEHEALHWHYTQKSLSLSLGK (SEQ ID NO: 68) |
| **Ab1 heavy chain IgG4 FALA and REW without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPE AAGGPSVFLFPPKPKDTLMISRTPEVTCVVVDVSQEDPEVQFNWYV DGVEVHNAKTKPREEQFNSTYRVVSVLTVLHRDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVEHEALHWHYTQKSLSLSLG (SEQ ID NO: 69) |
| **Ab1 heavy chain IgG4 LS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHSHYTQKSLSLSLGK (SEQ ID NO: 70) |
| **Ab1 heavy chain IgG4 LS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDTLMISRTPEVTCVVVDVSQEDPEVQFNWYVD |
| | GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHSHYTQKSLSLSLG (SEQ ID NO: 71) |
| **Ab1 heavy chain IgG4 STR and LS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHSHYTQKSLSLSLGK (SEQ ID NO: 72) |
| **Ab1 heavy chain IgG4 STR and LS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHSHYTQKSLSLSLG (SEQ ID NO: 73) |
| **Ab1 heavy chain IgG4 FALA and LS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS |
| | VVTVPSSSLGTKTYTCNVDHKPSNTKVDKRVESKYGPPCPSCPAPE AAGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVLHEALHSHYTQKSLSLSLGK (SEQ ID NO: 74) |
| **Ab1 heavy chain IgG4 FALA and LS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPE AAGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVLHEALHSHYTQKSLSLSLG (SEQ ID NO: 75) |
| **Ab1 heavy chain IgG4 QL** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDQLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHNHYTQKSLSLSLGK (SEQ ID NO: 76) |
| **Ab1 heavy chain IgG4 QL** | EVQLVESGGGLVQPGGSLRLPCAASGFTFYNYWITWIRQAPGKGLE WVANINQAGSEKSYLDAVKGRFTISRDNAKNSVYLQMNSLRVEDT |
| **without terminal lysine** | AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDQLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHNHYTQKSLSLSLG (SEQ ID NO: 77) |
| **Ab1 heavy chain IgG4 STR and QL** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDQLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHNHYTQKSLSLSLGK (SEQ ID NO: 78) |
| **Ab1 heavy chain IgG4 STR and QL without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDQLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHNHYTQKSLSLSLG (SEQ ID NO: 79) |
| **Ab1 heavy chain IgG4 FALA and QL** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPE AAGGPSVFLFPPKPKDQLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVLHEALHNHYTQKSLSLSLGK (SEQ ID NO: 80) |
| **Ab1 heavy chain IgG4 FALA and QL without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPE AAGGPSVFLFPPKPKDQLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVLHEALHNHYTQKSLSLSLG (SEQ ID NO: 81) |
| **Ab1 heavy chain IgG4 DHS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDTLMISRTPEVTCVVVDVSQEDPEVQFNWYVD GVEVHNAKTKPREEQFNSTYRVVSVLTVDHHDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHSHYTQKSLSLSLGK |
| | (SEQ ID NO: 82) |
| **Ab1 heavy chain IgG4 DHS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDTLMISRTPEVTCVVVDVSQEDPEVQFNWYVD GVEVHNAKTKPREEQFNSTYRVVSVLTVDHHDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHSHYTQKSLSLSLG (SEQ ID NO: 83) |
| **Ab1 heavy chain IgG4 STR and DHS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDTLMISRTPEVTCVVVDVSQEDPEVQFNWYVD GVEVHNAKTKPREEQFNSTYRVVSVLTVDHHDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHSHYTQKSLSLSLGK (SEQ ID NO: 84) |
| **Ab1 heavy chain IgG4 STR and DHS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPES TRGPSVFLFPPKPKDTLMISRTPEVTCVVVDVSQEDPEVQFNWYVD GVEVHNAKTKPREEQFNSTYRVVSVLTVDHHDWLNGKEYKCKVS NKGLPSSIEKTISKAKGQPREPQVYTLPPSQEEMTKNQVSLTCLVKG |
| | FYPSDIAVEWESNGQPENNYKTTPPVLDSDGSFFLYSRLTVDKSRW QEGNVFSCSVMHEALHSHYTQKSLSLSLG (SEQ ID NO: 85) |
| **Ab1 heavy chain IgG4 FALA and DHS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPE AAGGPSVFLFPPKPKDTLMISRTPEVTCVVVDVSQEDPEVQFNWYV DGVEVHNAKTKPREEQFNSTYRVVSVLTVDHHDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHSHYTQKSLSLSLGK (SEQ ID NO: 86) |
| **Ab1 heavy chain IgG4 FALA and DHS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPE AAGGPSVFLFPPKPKDTLMISRTPEVTCVVVDVSQEDPEVQFNWYV DGVEVHNAKTKPREEQFNSTYRVVSVLTVDHHDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHSHYTQKSLSLSLG (SEQ ID NO: 87) |
| **Ab1 heavy chain IgG4 S228P and STR** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDTLMISRTPEVTCVVVDVSQEDPEVQFNWYVD |
| | GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLGK (SEQ ID NO: 88) |
| **Ab1 heavy chain IgG4 S228P and STR without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLG (SEQ ID NO: 89) |
| **Ab1 heavy chain IgG4 S228P and FALA** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLGK (SEQ ID NO: 90) |
| **Ab1 heavy chain IgG4 S228P and FALA without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS |
| | VVTVPSSSLGTKTYTCNVDHKPSNTKVDKRVESKYGPPCPPCPAPE AAGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLG (SEQ ID NO: 91) |
| **Ab1 heavy chain IgG4 S228P and YTE** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLYITREPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLGK (SEQ ID NO: 92) |
| **Ab1 heavy chain IgG4 S228P and YTE without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLYITREPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLG (SEQ ID NO: 93) |
| **Ab1 heavy chain IgG4** | EVQLVESGGGLVQPGGSLRLPCAASGFTFYNYWITWIRQAPGKGLE WVANINQAGSEKSYLDAVKGRFTISRDNAKNSVYLQMNSLRVEDT |
| **S228P and STR and YTE** | AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDTLYITREPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLGK (SEQ ID NO: 94) |
| **Ab1 heavy chain IgG4 S228P and STR and YTE without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDTLYITREPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLG (SEQ ID NO: 95) |
| **Ab1 heavy chain IgG4 S228P and FALA and YTE** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDTLYITREPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLGK (SEQ ID NO: 96) |
| **Ab1 heavy chain IgG4 S228P and FALA and YTE without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDTLYITREPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHNHYTQKSLSLSLG (SEQ ID NO: 97) |
| **Ab1 heavy chain IgG4 S228P and REW** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLMISRTPEVTCVVVDVSQEDPEVQFNWYVD GVEVHNAKTKPREEQFNSTYRVVSVLTVLHRDWLNGKEYKCKVSN KGLPSSIEKTISKAKGQPREPQVYTLPPSQEEMTKNQVSLTCLVKGF YPSDIAVEWESNGQPENNYKTTPPVLDSDGSFFLYSRLTVDKSRWQ EGNVFSCSVEHEALHWHYTQKSLSLSLGK (SEQ ID NO: 98) |
| **Ab1 heavy chain IgG4 S228P and REW without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLMISRTPEVTCVVVDVSQEDPEVQFNWYVD GVEVHNAKTKPREEQFNSTYRVVSVLTVLHRDWLNGKEYKCKVSN KGLPSSIEKTISKAKGQPREPQVYTLPPSQEEMTKNQVSLTCLVKGF YPSDIAVEWESNGQPENNYKTTPPVLDSDGSFFLYSRLTVDKSRWQ EGNVFSCSVEHEALHWHYTQKSLSLSLG |
| | (SEQ ID NO: 99) |
| **Ab1 heavy chain IgG4 S228P and STR and REW** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDTLMISRTPEVTCVVVDVSQEDPEVQFNWYVD GVEVHNAKTKPREEQFNSTYRVVSVLTVLHRDWLNGKEYKCKVSN KGLPSSIEKTISKAKGQPREPQVYTLPPSQEEMTKNQVSLTCLVKGF YPSDIAVEWESNGQPENNYKTTPPVLDSDGSFFLYSRLTVDKSRWQ EGNVFSCSVEHEALHWHYTQKSLSLSLGK (SEQ ID NO: 100) |
| **Ab1 heavy chain IgG4 S228P and STR and REW without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDTLMISRTPEVTCVVVDVSQEDPEVQFNWYVD GVEVHNAKTKPREEQFNSTYRVVSVLTVLHRDWLNGKEYKCKVSN KGLPSSIEKTISKAKGQPREPQVYTLPPSQEEMTKNQVSLTCLVKGF YPSDIAVEWESNGQPENNYKTTPPVLDSDGSFFLYSRLTVDKSRWQ EGNVFSCSVEHEALHWHYTQKSLSLSLG (SEQ ID NO: 101) |
| **Ab1 heavy chain IgG4 S228P and FALA and REW** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDTLMISRTPEVTCVVVDVSQEDPEVQFNWYV DGVEVHNAKTKPREEQFNSTYRVVSVLTVLHRDWLNGKEYKCKVS NKGLPSSIEKTISKAKGQPREPQVYTLPPSQEEMTKNQVSLTCLVKG |
| | FYPSDIAVEWESNGQPENNYKTTPPVLDSDGSFFLYSRLTVDKSRW QEGNVFSCSVEHEALHWHYTQKSLSLSLGK (SEQ ID NO: 102) |
| **Ab1 heavy chain IgG4 S228P and FALA and REW without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDTLMISRTPEVTCVVVDVSQEDPEVQFNWYV DGVEVHNAKTKPREEQFNSTYRVVSVLTVLHRDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVEHEALHWHYTQKSLSLSLG (SEQ ID NO: 103) |
| **Ab1 heavy chain IgG4 S228P and LS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHSHYTQKSLSLSLGK (SEQ ID NO: 104) |
| **Ab1 heavy chain IgG4 S228P and LS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLMISRTPEVTCVVVDVSQEDPEVQFNWYVD |
| | GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHSHYTQKSLSLSLG (SEQ ID NO: 105) |
| **Ab1 heavy chain IgG4 S228P and STR and LS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHSHYTQKSLSLSLGK (SEQ ID NO: 106) |
| **Ab1 heavy chain IgG4 S228P and STR and LS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHSHYTQKSLSLSLG (SEQ ID NO: 107) |
| **Ab1 heavy chain IgG4 S228P and FALA and LS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS |
| | VVTVPSSSLGTKTYTCNVDHKPSNTKVDKRVESKYGPPCPPCPAPE AAGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVLHEALHSHYTQKSLSLSLGK (SEQ ID NO: 108) |
| **Ab1 heavy chain IgG4 S228P and FALA and LS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDTLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVLHEALHSHYTQKSLSLSLG (SEQ ID NO: 109) |
| **Ab1 heavy chain IgG4 S228P and QL** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDQLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHNHYTQKSLSLSLGK (SEQ ID NO: 110) |
| **Ab1 heavy chain IgG4 S228P and QL** | EVQLVESGGGLVQPGGSLRLPCAASGFTFYNYWITWIRQAPGKGLE WVANINQAGSEKSYLDAVKGRFTISRDNAKNSVYLQMNSLRVEDT |
| **without terminal lysine** | AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDQLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHNHYTQKSLSLSLG (SEQ ID NO: 111) |
| **Ab1 heavy chain IgG4 S228P and STR and QL** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDQLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHNHYTQKSLSLSLGK (SEQ ID NO: 112) |
| **Ab1 heavy chain IgG4 S228P and STR and QL without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDQLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVLHEALHNHYTQKSLSLSLG (SEQ ID NO: 113) |
| **Ab1 heavy chain IgG4 S228P and FALA and QL** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDQLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVLHEALHNHYTQKSLSLSLGK (SEQ ID NO: 114) |
| **Ab1 heavy chain IgG4 S228P and FALA and QL without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDQLMISRTPEVTCVVVDVSQEDPEVQFNWYV DGVEVHNAKTKPREEQFNSTYRVVSVLTVLHQDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVLHEALHNHYTQKSLSLSLG (SEQ ID NO: 115) |
| **Ab1 heavy chain IgG4 S228P and DHS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLMISRTPEVTCVVVDVSQEDPEVQFNWYVD GVEVHNAKTKPREEQFNSTYRVVSVLTVDHHDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHSHYTQKSLSLSLGK |
| | (SEQ ID NO: 116) |
| **Ab1 heavy chain IgG4 S228P and DHS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLMISRTPEVTCVVVDVSQEDPEVQFNWYVD GVEVHNAKTKPREEQFNSTYRVVSVLTVDHHDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHSHYTQKSLSLSLG (SEQ ID NO: 117) |
| **Ab1 heavy chain IgG4 S228P and STR and DHS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDTLMISRTPEVTCVVVDVSQEDPEVQFNWYVD GVEVHNAKTKPREEQFNSTYRVVSVLTVDHHDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHSHYTQKSLSLSLGK (SEQ ID NO: 118) |
| **Ab1 heavy chain IgG4 S228P and STR and DHS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPES TRGPSVFLFPPKPKDTLMISRTPEVTCVVVDVSQEDPEVQFNWYVD GVEVHNAKTKPREEQFNSTYRVVSVLTVDHHDWLNGKEYKCKVS NKGLPSSIEKTISKAKGQPREPQVYTLPPSQEEMTKNQVSLTCLVKG |
| | FYPSDIAVEWESNGQPENNYKTTPPVLDSDGSFFLYSRLTVDKSRW QEGNVFSCSVMHEALHSHYTQKSLSLSLG (SEQ ID NO: 119) |
| **Ab1 heavy chain IgG4 S228P and FALA and DHS** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDTLMISRTPEVTCVVVDVSQEDPEVQFNWYV DGVEVHNAKTKPREEQFNSTYRVVSVLTVDHHDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHSHYTQKSLSLSLGK (SEQ ID NO: 120) |
| **Ab1 heavy chain IgG4 S228P and FALA and DHS without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPE AAGGPSVFLFPPKPKDTLMISRTPEVTCVVVDVSQEDPEVQFNWYV DGVEVHNAKTKPREEQFNSTYRVVSVLTVDHHDWLNGKEYKCKV SNKGLPSSIEKTISKAKGQPREPQVYTLPPSQEEMTKNQVSLTCLVK GFYPSDIAVEWESNGQPENNYKTTPPVLDSDGSFFLYSRLTVDKSR WQEGNVFSCSVMHEALHSHYTQKSLSLSLG (SEQ ID NO: 121) |
| **Ab1 heavy chain IgG4** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDTLMISRTPEVTCVVVDVSQEDPEVQFNWYVD |
| | GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLGK (SEQ ID NO: 122) |
| **Ab1 heavy chain IgG4 without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPSCPAPEF LGGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLG (SEQ ID NO: 123) |
| **Ab1 heavy chain IgG4 S228P** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS VVTVPSSSLGTKTYTCNVDHKPSNTKVDKRVESKYGPPCPPCPAPEF LGGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLGK (SEQ ID NO: 124) |
| **Ab1 heavy chain IgG4 S228P without terminal lysine** | EVQLVESGGGLVQPGGSLRLPCAASGFTFYNYWITWIRQAPGKGLE WVANINQAGSEKSYLDAVKGRFTISRDNAKNSVYLQMNSLRVEDT AVYYCAGGFRAFDIWGQGTMVTVSSASTKGPSVFPLAPCSRSTSES TAALGCLVKDYFPEPVTVSWNSGALTSGVHTFPAVLQSSGLYSLSS |
| | VVTVPSSSLGTKTYTCNVDHKPSNTKVDKRVESKYGPPCPPCPAPEF LGGPSVFLFPPKPKDTLMISRTPEVTCVVVDVSQEDPEVQFNWYVD GVEVHNAKTKPREEQFNSTYRVVSVLTVLHQDWLNGKEYKCKVS NKGLPSSIEKTISKAKGQPREPQVYTLPPSQEEMTKNQVSLTCLVKG FYPSDIAVEWESNGQPENNYKTTPPVLDSDGSFFLYSRLTVDKSRW QEGNVFSCSVMHEALHNHYTQKSLSLSLG (SEQ ID NO: 125) |

Embodiments of the anti-CD79b antibody may comprise an IgG₁ heavy chain comprising amino acid sequences according to SEQ ID NOs: 9, 13 and 20-53. In embodiments, the anti-CD79b antibody may comprise a wild type IgG₁ heavy chain comprising amino acid sequences according to SEQ ID NO 9 (with terminal lysine) and SEQ ID NO 13 (without terminal lysine). In embodiments, the IgG1 heavy chain comprises Fc silencing mutations selected from L234A, L235A (LALA) or L234S, L235T, G236R (STR). In embodiments, the anti-CD79b antibody comprises an IgG₁ heavy chain comprising the mutations L234A, L235A (LALA) with or without terminal lysine. In embodiments, the anti-CD79b antibody comprises an IgG₁ heavy chain comprising the mutations L234S, L235T, G236R (STR) with or without terminal lysine. In embodiments, the anti-CD79b antibody may comprise an heavy chain IgG1 comprising half-life extension mutations selected from M252Y, S254T, T256E (YTE); Q311R, M428E, N434W (REW); M428L, N434S (LS); L309D, Q311H, N434S (DHS); or T250Q, M428L (QL). In embodiments, the anti-CD79b antibody comprises an IgG1 heavy chain comprising M252Y, S254T, T256E (YTE) with or without terminal lysine. In embodiments, the anti-CD79b antibody comprises an IgG1 heavy chain comprising Q311R, M428E, N434W (REW) with or without terminal lysine. In embodiments, the anti-CD79b antibody comprises an IgG₁ heavy chain comprising M428L, N434S (LS) with or without terminal lysine. In embodiments, the anti-CD79b antibody comprises an IgG1 heavy chain comprising L309D, Q311H, N434S (DHS) with or without terminal lysine. In embodiments, the anti-CD79b antibody comprises an IgG1 heavy chain comprising T250Q, M428L (QL) with or without terminal lysine. In embodiments, the anti-CD79b antibody may comprise both Fc silencing mutations and half-life extension mutations. In embodiments, the anti-CD79b antibody comprises an IgG₁ heavy chain comprising the mutations L234A, L235A (LALA) and M252Y, S254T, T256E (YTE) with or without terminal lysine. In embodiments, the anti-CD79b antibody comprises an IgG1 heavy chain comprising the mutations L234S, L235T, G236R (STR) and M252Y, S254T, T256E (YTE) with or without terminal lysine. In embodiments, the anti-CD79b antibody comprises an IgG1 heavy chain comprising the mutations L234A, L235A (LALA) and Q311R, M428E, N434W (REW) with or without terminal lysine. In embodiments, the anti-CD79b antibody comprises an IgG1 heavy chain comprising the mutations L234S, L235T, G236R (STR) and Q311R, M428E, N434W (REW) with or without terminal lysine. In embodiments, the anti-CD79b antibody comprises an IgG₁ heavy chain comprising the mutations L234A, L235A (LALA) and M428L, N434S (LS) with or without terminal lysine. In embodiments, the anti-CD79b antibody comprises an IgG₁ heavy chain comprising the mutations L234S, L235T, G236R (STR) and M428L, N434S (LS) with or without terminal lysine. In embodiments, the anti-CD79b antibody comprises an IgG₁ heavy chain comprising the mutations L234A, L235A (LALA) and L309D, Q311H, N434S (DHS) with or without terminal lysine. In embodiments, the anti-CD79b antibody comprises an IgG1 heavy chain comprising the mutations L234S, L235T, G236R (STR) and L309D, Q311H, N434S (DHS) with or without terminal lysine. In embodiments, the anti-CD79b antibody comprises an IgG1 heavy chain comprising the mutations L234A, L235A (LALA) and T250Q, M428L (QL) with or without terminal lysine. In embodiments, the anti-CD79b antibody comprises an IgG1 heavy chain comprising the mutations L234S, L235T, G236R (STR) and T250Q, M428L (QL) with or without terminal lysine.

Embodiments of the anti-CD79b antibody may comprise an IgG4 heavy chain comprising amino acid sequences according to SEQ ID NOs: 54-125. In embodiments, the anti-CD79b antibody may comprise a wild type IgG4 heavy chain comprising amino acid sequences according to SEQ ID NO 122 (with terminal lysine) and SEQ ID NO 123 (without terminal lysine). In embodiments, the anti-CD79b antibody may comprise the IgG4 heavy chain further comprising an S228P mutation according to SEQ ID NO 124 (with terminal lysine) and SEQ ID NO 125 (without terminal lysine). In embodiments, the IgG4 heavy chain comprises Fc silencing mutations selected from F234A, L235A (FALA) or L234S, L235T, G236R (STR). In embodiments, the anti-CD79b antibody comprises an IgG4 heavy chain comprising the mutations F234A, L235A (FALA) with or without terminal lysine and with or without an S228P mutation. In embodiments, the anti-CD79b antibody comprises an IgG4 heavy chain comprising the mutations L234S, L235T, G236R (STR) with or without terminal lysine and with or without an S228P mutation. In embodiments, the anti-CD79b antibody may comprise an IgG4 heavy chain comprising half-life extension mutations selected from M252Y, S254T, T256E (YTE); Q311R, M428E, N434W (REW); M428L, N434S (LS); L309D, Q311H, N434S (DHS); or T250Q, M428L (QL). In embodiments, the anti-CD79b antibody comprises an IgG4 heavy chain comprising M252Y, S254T, T256E (YTE) with or without terminal lysine and with or without an S228P mutation. In embodiments, the anti-CD79b antibody comprises an IgG4 heavy chain comprising Q311R, M428E, N434W (REW) with or without terminal lysine and with or without an S228P mutation. In embodiments, the anti-CD79b antibody comprises an IgG4 heavy chain comprising M428L, N434S (LS) with or without terminal lysine and with or without an S228P mutation. In embodiments, the anti-CD79b antibody comprises an IgG4 heavy chain comprising L309D, Q311H, N434S (DHS) with or without terminal lysine and with or without an S228P mutation. In embodiments, the anti-CD79b antibody comprises an IgG4 heavy chain comprising T250Q, M428L (QL) with or without terminal lysine and with or without an S228P mutation. In embodiments, the anti-CD79b antibody may comprise both Fc silencing mutations and half-life extension mutations. In embodiments, the anti-CD79b antibody comprises an IgG4 heavy chain comprising the mutations F234A, L235A (FALA) and M252Y, S254T, T256E (YTE) with or without terminal lysine and with or without an S228P mutation. In embodiments, the anti-CD79b antibody comprises an IgG4 heavy chain comprising the mutations L234S, L235T, G236R (STR) and M252Y, S254T, T256E (YTE) with or without terminal lysine and with or without an S228P mutation. In embodiments, the anti-CD79b antibody comprises an IgG4 heavy chain comprising the mutations F234A, L235A (FALA) and Q311R, M428E, N434W (REW) with or without terminal lysine and with or without an S228P mutation. In embodiments, the anti-CD79b antibody comprises an IgG4 heavy chain comprising the mutations L234S, L235T, G236R (STR) and Q311R, M428E, N434W (REW) with or without terminal lysine and with or without an S228P mutation. In embodiments, the anti-CD79b antibody comprises an IgG4 heavy chain comprising the mutations F234A, L235A (FALA) and M428L, N434S (LS) with or without terminal lysine and with or without an S228P mutation. In embodiments, the anti-CD79b antibody comprises an IgG4 heavy chain comprising the mutations L234S, L235T, G236R (STR) and M428L, N434S (LS) with or without terminal lysine and with or without an S228P mutation. In embodiments, the anti-CD79b antibody comprises an IgG4 heavy chain comprising the mutations F234A, L235A (FALA) and L309D, Q311H, N434S (DHS) with or without terminal lysine and with or without an S228P mutation. In embodiments, the anti-CD79b antibody comprises an IgG4 heavy chain comprising the mutations L234S, L235T, G236R (STR) and L309D, Q311H, N434S (DHS) with or without terminal lysine and with or without an S228P mutation. In embodiments, the anti-CD79b antibody comprises an IgG4 heavy chain comprising the mutations F234A, L235A (FALA) and T250Q, M428L (QL) with or without terminal lysine and with or without an S228P mutation. In embodiments, the anti-CD79b antibody comprises an IgG4 heavy chain comprising the mutations L234S, L235T, G236R (STR) and T250Q, M428L (QL) with or without terminal lysine and with or without an S228P mutation.

In certain embodiments, the anti-CD79b antibody comprises a heavy chain comprising the amino acid sequence encoded by the nucleotide sequence of SEQ ID NO:11; and/or a light chain comprising the amino acid sequence encoded by the nucleotide sequence of SEQ ID NO: 12.

The present disclosure further provides polynucleotides encoding the anti-CD79b antibody disclosed herein or one or more chains thereof. In certain embodiments, the present disclosure provides a set of polynucleotides encoding the anti-CD79b antibody disclosed herein. In certain embodiments, the polynucleotide encodes a heavy chain or a light chain of the anti-CD79b antibody disclosed herein. In certain embodiments, the polynucleotide encodes a heavy chain and a light chain of the anti-CD79b antibody disclosed herein. In certain embodiments, the set of polynucleotides comprise a polynucleotide encoding a heavy chain of the anti-CD79b antibody disclosed herein and a polynucleotide encoding a light chain of the anti-CD79b antibody disclosed herein.

The polynucleotides disclosed herein encompass polynucleotides that comprise only coding sequences for the anti-CD79b antibody or a portion thereof, as well as polynucleotides that comprise additional coding and/or non-coding sequences. The polynucleotides disclosed herein can be in the form of RNA or DNA (*e.g.,* cDNA, genomic DNA, and synthetic DNA), whether double-stranded or single-stranded.

In certain embodiments, the polynucleotide encodes a VH or a VL of the anti-CD79b antibody disclosed herein. In certain embodiments, the polynucleotide encodes a VH and a VL of the anti-CD79b antibody disclosed herein. In certain embodiments, the set of polynucleotides comprise a polynucleotide encoding a VH of the anti-CD79b antibody disclosed herein and a polynucleotide encoding a VL of the anti-CD79b antibody disclosed herein.

In certain embodiments, provided herein is a vector or a set of vectors comprising a polynucleotide or a set of polynucleotides disclosed herein, where the polynucleotide or set of polynucleotides encodes the anti-CD79b antibody disclosed herein or a portion thereof.

The polynucleotides may be present in whole cells, in a cell lysate, or in a partially purified or substantially pure form. A polynucleotide is isolated or rendered substantially pure when separated from other cellular components or other contaminants, *e.g*., other cellular nucleic acids or proteins, by standard techniques known in the art, including but not limited to alkaline/SDS treatment, CsCl banding, column chromatography, and agarose gel electrophoresis.

Polynucleotides of embodiments described herein can be obtained using standard molecular biology techniques. For antibodies expressed by hybridomas, cDNAs encoding the light and heavy chains of the antibody can be obtained by standard PCR amplification or cDNA cloning techniques. For antibodies obtained from an immunoglobulin gene library (*e.g.,* using phage display techniques), polynucleotides encoding the antibody can be recovered from the library.

In certain embodiments, disclosed herein is a pharmaceutical composition, comprising an anti-CD79b antibody disclosed herein and a pharmaceutically acceptable carrier.

A variable region (*e.g*., VH and VL) is a portion of the light or heavy chains of an antibody that is primarily responsible for specific binding of each antibody to its particular antigen. Each variable region comprises framework regions (FRs) and complementarity determining regions (CDRs). FRs are less variable stretches of the variable region and are separated by CDRs, which are shorter regions of greater variability.

### 1.2 Topoisomerase 1 Inhibitors (Topli)

Topoisomerase 1 (Top1) are enzymes that remove supercoils formed during DNA replication. Top1 inhibitors (Top1i) can bind and stabilize Top1-DNA complexes, thus inducing DNA strand breakage and apoptosis.

Disclosed herein is a Top1i drug according to structural formula (I), which may be purposed for targeted delivery to cells by conjugation to an anti-CD79b antibody.

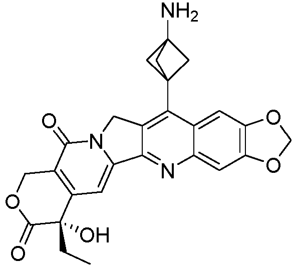

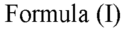

In embodiments, the Top1i drug is (7*S*)-14-(3-aminobicyclo[1.1.1]pentan-1-yl)-7-ethyl-7-hydroxy-2*H*,10*H*-[1,3]dioxolo[4,5-*g*]pyrano[3',4':6,7]indolizino[1,2-*b*]quinoline-8,11(7*H*,13*H*)-dione.

In certain embodiments, the Top1i drug as contemplated herein may be conjugated to an antibody in an ADC as shown in structural formula (II): 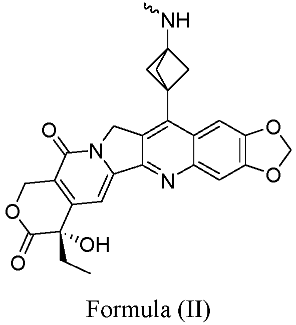 wherein 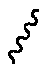 represents the point of attachment of a linker to the Top1i drug.

### 1.2.1 Linker Drug LD1 (Structural Formula (III))

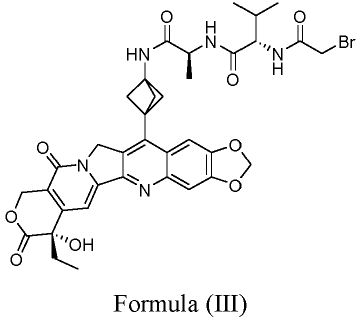

In certain embodiments, a Top1i linker drug (LD1) is a compound according to formula (III). In certain embodiments, LD1 is (2S)-2-(2-bromoacetamido)-*N*-[(2S)-1-({3-[(7*S*)-7-ethyl-7-hydroxy-8,11-dioxo-7,8,11,13-tetrahydro-2*H*,10*H*-[1,3]dioxolo[4,5-*g*]pyrano[3',4':6,7]indolizino[1,2-*b*]quinolin-14-yl]bicyclo[1.1.1]pentan-1-yl}amino)-1-oxopropan-2-yl]-3-methylbutanamide.

### 1.3 Anti-CD79b Top1i ADCs

In certain embodiments, the Top1i drug disclosed herein (*i.e.,* formula (I)) may be conjugated to an anti-CD79b antibody disclosed herein to form an anti-CD79b Top1i ADC. ADCs can selectively deliver one or more drug moiety(s) to target tissues, such as CD79b expressing tumors. Thus, in certain embodiments, the present disclosure provides anti-CD79b TOP1i ADCs for therapeutic use in the treatment of B-cell malignancies.

In certain embodiments, the Top1i drug is conjugated to the anti-CD79b antibody by way of a linker moiety. In certain embodiments, the linker connects the Top1i drug to the anti-CD79b antibody by forming a covalent linkage to the Top1i drug at one location and a covalent linkage to the antibody at another. In certain embodiments, the covalent linkages are formed by reactions between functional groups on the linker and functional groups on the Top1i drug and the anti-CD79b antibody. In certain embodiments, the anti-CD79b Top1i ADC of the present disclosure comprises an anti-CD79b antibody conjugated to the Top1i linker drug of formula (III).

### 1.3.1 Number of Linked Drugs

In certain embodiments, the anti-CD79b Top1i ADCs disclosed herein comprise drug molecules linked to antibody moieties in various stoichiometric molar ratios depending on the configuration of the antibody and, at least in part, the method used to effect conjugation.

The terms "drug load" or "drug loading" refer to the number of drug molecules per antibody in an individual ADC molecule.

The number of cytotoxic and/or cytostatic agents linked to the antigen binding moiety of an anti-CD79b Top1i ADC can vary (called the "drug-to-antibody ratio," or "DAR"). The DAR is the average number of drugs linked to each antibody in the composition. The number of Top1i drugs linked to an anti-CD79b antibody to provide embodiments of the CD79b Top1i ADCs described herein may vary and will be limited by the number of available attachment sites on the anti-CD79b antibody. As contemplated for the anti-CD79b Top1i ADCs described herein, a linker links a single Top1i drug to the anti-CD79b antibody in an anti-CD79b Top1i ADC. In certain embodiments, the anti-CD79b Top1i ADCs have an n of in the range of from 1 to 8. In certain embodiments, the anti-CD79b Top1i ADCs have an n of 1, 2, 3, 4, 5, 6, 7, or 8. In certain embodiments, the drug loading comprises 1 drug molecule, 2 drug molecules, 3 drug molecules, 4 drug molecules, 5 drug molecules, 6 drug molecules, 7 drug molecules, or 8 drug molecules. In embodiments, the anti-CD79b Top1i ADCs described herein may have a DAR in the range of about n = 1-8, or 1-6. In a specific embodiment, the anti-CD79b Top1i ADC described herein has a DAR of 1-6 or about 6.

### 1.3.2 Exemplary ADCs

In certain embodiments, an anti-CD79b Top1i ADC of formula (IV) is provided: 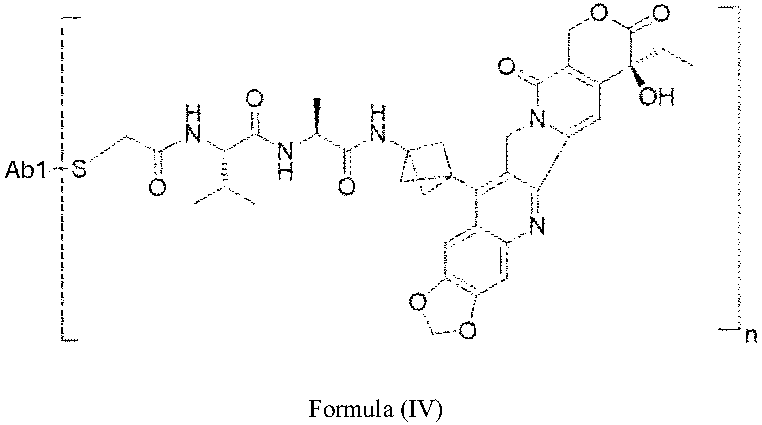 wherein antibody Ab1 is an anti-CD79b antibody comprising a VH-CDR1 comprising the amino acid sequence of SEQ ID NO: 1, a VH-CDR2 comprising the amino acid sequence of SEQ ID NO: 2, a VH-CDR3 comprising the amino acid sequence of SEQ ID NO: 3, a VL-CDR1 comprising the amino acid sequence of SEQ ID NO: 4, a VL-CDR2 comprising the amino acid sequence of SEQ ID NO: 5, and a VL-CDR3 comprising the amino acid sequence of SEQ ID NO: 6. In certain embodiments, the anti-CD79b antibody comprises a VH-CDR1, a VH-CDR2, and a VH-CDR3 as set forth in a VH comprising the amino acid sequence of SEQ ID NO: 7; and a VL-CDR1, a VL-CDR2, and a VL-CDR3 as set forth in a VL comprising the amino acid sequence of SEQ ID NO: 8. In certain embodiments, the anti-CD79b antibody comprises a VH comprising the amino acid sequence of SEQ ID NO: 7 and a VL comprising the amino acid sequence of SEQ ID NO: 8. In certain embodiments, the anti-CD79b antibody comprises a heavy chain comprising the amino acid sequence of SEQ ID NO: 9 or SEQ ID NO: 13 and a light chain comprising the amino acid sequence of SEQ ID NO: 10. In certain embodiments, conjugation of the linker-drug to the anti-CD79b antibody is via a linkage formed with a sulfhydryl group of a cysteine residue of the anti-CD79b antibody. In embodiments, n has a value of 1, 2, 3, 4, 5, 6, 7, or 8.

As used herein, "ADC-1" is an anti-CD79b Top1i ADC composition comprising anti-CD79b ADCs according to structural formula (IV), wherein the Ab1 comprises two heavy chains each comprising the amino acid sequence of SEQ ID NO: 9 or SEQ ID NO: 13 and two light chains each comprising the amino acid sequence of SEQ ID NO: 10. A method for making ADC-1 is described in the Examples below. In certain embodiments, ADC-1 is an anti-CD79b ADC composition comprising a plurality of ADC-1 species with two or more different values of n.

The anti-CD79b ADCs of the present disclosure may be provided as a composition suitable for administration to a patient. In certain embodiments, the anti-CD79b ADC composition is a pharmaceutical composition, comprising an anti-CD79b ADC of the present disclosure and a pharmaceutically acceptable carrier. A given formulation of the anti-CD79b ADCs disclosed herein may comprise a distribution of antibodies having differing values of *n.*

### 1.4 Methods of Use

The present disclosure further provides methods for treating B-cell malignancies comprising administering to a subject in need thereof a therapeutically effective amount of an ADC of formula (IV). In certain embodiments, the B-cell malignancy is B-NHL, including but not limited to DLBCL, BL, MCL, and FL.

The terms "patient", "subject", "individual" and the like refer to humans.

The terms "treat," "treating," and "treatment," as used herein, refer to a method of alleviating or abrogating a disease and/or its attendant symptoms.

The phrase "therapeutically effective amount" refers to an amount of an ADC sufficient to prevent the development of or to alleviate to some extent one or more of the symptoms of the condition or disorder being treated when administered for treatment in a particular subject or subject population.

### 2. EXAMPLES

The following Examples, which highlight certain features and properties of the exemplary embodiments of the ADCs, antibodies, and Top1i as described herein, are provided for purposes of illustration.

### 2.1 Example 1: Screening and Preparation of Ab1

Anti-CD79b antibodies were derived from recombinant fully human immunoglobulin G1 and were generated using AlivaMab^{®} (Ablexis) mice. The CD79b monoclonal antibody candidate was identified based on expression, *in vitro* binding potency to human CD79b, lack of binding to human CD79a, and potency against B-NHL cancer cells when conjugated to Topli payload.

The sequence information of anti-CD79b Ab1 is provided in **Table 1.** Ab1 was expressed in Chinese hamster ovary (CHO) cells with the heavy and light chain sequences of SEQ ID NOs: 9 or 13 and 10, respectively. Ab1 was isolated and purified prior to conjugation.

### 2.2 Example 2: Procedure for Conjugation to Produce ADC-1

### Conjugation Step 1

A solution of antibody (17.9 mg/mL) at 4 °C in DPBS (Dulbecco's Phosphate Buffer Saline with 5 mM ethylenediaminetetraacetic acid (EDTA), pH 7.2) was treated with tris (2-carboxyethyl) phosphine hydrochloride (TCEP, 10 mM, 5 molar equivalents, Bond-Breaker^{®}, Thermo Scientific^{®}) at 4 °C for 20 hours. Following incubation, 1 M borate buffer (10% v/v, pH 8) was added to adjust the pH, followed by addition of excess (10 equivalents) of a linker drug according to structural formula (III) (in10 mM dimethylacetamide (DMA) solution).

The reaction mixture was kept at room temperature for 1 hour in the dark. The resulting ADC was purified. The resulting ADC solution was filtered and stored in an amber tube at 4 °C, DAR = 6.05.

**DAR Determination.** DAR was determined by LC-MS or HIC (hydrophobic interaction chromatography). LC-MS analysis was performed using an Agilent 1100 HPLC system interfaced to an Agilent LC/MSD TOF (Time-of-Flight) 6220 ESI mass spectrometer. The ADC was reduced with 5 mM (final concentration) Bond-Breaker^{®} TCEP solution (Thermo Scientific^{®}, Rockford, IL), loaded onto a protein microtrap (Michrom BioResources) desalting cartridge, and eluted with a gradient of 10% B to 75% B in 0.2 minutes at ambient temperature. Mobile phase A was H₂O with 0.1% formic acid (FA), mobile phase B was acetonitrile with 0.1% FA, and the flow rate was 0.2 mL/minute. Electrospray-ionization time-of-flight mass spectra of the co-eluting light and heavy chains were acquired using Agilent MassHunter acquisition software. The extracted intensity vs. m/z spectrum was deconvoluted using the Maximum Entropy feature of MassHunter software to determine the mass of each reduced antibody fragment. DAR was calculated from the deconvoluted spectrum by summing intensities of the naked and modified peaks for the light chain and heavy chain, normalized by multiplying intensity by the number of drugs attached. The summed, normalized intensities were divided by the sum of the intensities, and the summing results for two light chains and two heavy chains produced a final average DAR value for the full ADC.

Peptide mapping was carried out to determine modifications or deletions to the amino acids present in the heavy and light chains of Ab1. Following denaturation, reduction, and alkylation, Ab1 was subjected to digestion using the proteolytic enzyme, Lys-C, which cleaves peptide bonds on the C-terminal end of lysine. Digested samples were analyzed using reverse-phase high-performance liquid chromatography (HPLC).

Analysis of two batches of Ab1 peptide fragments following proteolytic digestion showed that two species of the heavy chain were present. Total ion current (TIC) chromatograms were generated. Only a small percentage (2.9 percent) of the heavy chain of Ab1 consisted of heavy chains with a C-terminal lysine, as is shown in SEQ ID NO: 9. The predominant species of the heavy chain in Ab1 lacked the C-terminal lysine, as is shown in SEQ ID NO: 13.

### 2.3 Example 3: Binding Affinity of ADC-1

Polatuzumab vedotin-piiq is a CD79b-directed ADC used to treat B-NHL.

ADC-1 generated by the procedures of Example 3 has the following structure: 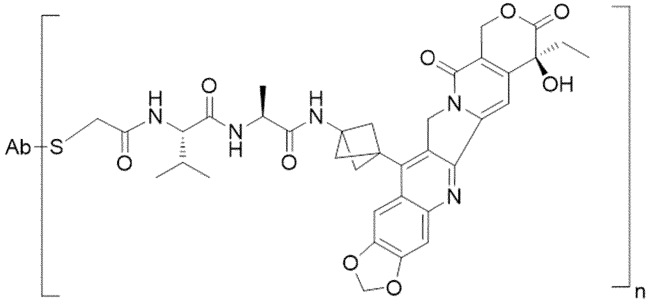 where n is 6, and Ab is anti-CD79b antibody Ab1.

Binding affinity of ADC-1 and polatuzumab vedotin-piiq to human CD79b was measured. The binding ligand used in the assay was recombinant human CD79b having the amino acid sequence of Ala29-Asp159 of human CD79b, which was fused with a 6-His tag at the C terminus. **Table 3** provides the measured association rate (kₐ), dissociation rate (k_{d}), and equilibrium dissociation constant (K_{D}).

**Table 3: Binding Affinity**

| **Ligands** | **Human CD79b (AA 29-159)-6-His** | | |
|---|---|---|---|
| | **kₐ (1/Ms)** | **k_{d} (1/s)** | **K_{D} (M)** |
| **ADC-1** | 1.7×10⁵ | 6.8×10⁻⁴ | 4.0×10⁻⁹ |
| **Polatuzumab vedotin-piiq** | 1.7×10⁷ | 8.7×10⁻³ | 5.0×10⁻⁹ |

### 2.4 Example 4: In Vitro Cytotoxicity

*In vitro* cytotoxicity of ADC-1 and polatuzumab vedotin-piiq was measured in a number of B-NHL cell lines, including DoHH2, HBL-1, JEKO, REC-1, Ramos, and SuDHL-4. SuDHL-4 and DoHH2 are DLBCL-germinal center B cell like (GCB) cell lines; HBL-1 is a DLBCL-activated B cell like (ABC) cell line; Ramos is a BL cell line; and JEKO and REC-1 are MCL cell lines. **Table 4** shows the *in vitro* cytotoxicity results.

**Table 4: In Vitro Cytotoxicity (EC₅₀, nM)**

| | **DoHH2** | **HBL-1** | **JEKO** | **REC-1** | **Ramos** | **SuDHL-4** |
|---|---|---|---|---|---|---|
| **ADC-1** | 0.30 | 0.73 | 1.58 | 0.64 | 0.78 | 1.40 |
| **Polatuzumab vedotin-piiq** | 1.30 | 1.14 | 0.90 | 3.19 | 0.49 | 1.24 |

### 2.5 Example 5: In Vivo Cytotoxicity

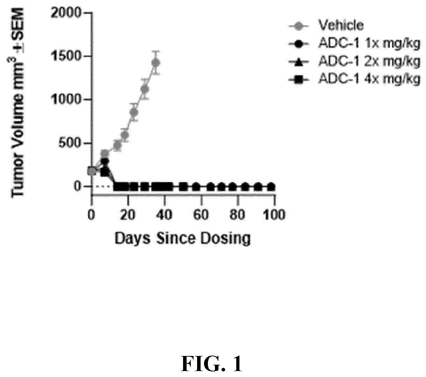

*In vivo* growth inhibition of DLBCL ABC CDX tumors (U-2932) treated with ADC-1 was measured at a number of doses of ADC-1, including the initial dose level (1x), two-times the initial dose level (2x), and four-times the initial dose level (4x). Administration of each dose of ADC-1 was as a single bolus intraperitoneally on day zero. ADC-1 induced statistically significant tumor growth inhibition and tumor growth delay compared to vehicle controls. As shown in **FIG. 1****,** ADC-1 administration to xenografted DLBCL ABC CDX tumors significantly reduced tumor volume over a period of 110 days at all dose levels tested. CRs of 100% were observed at all dose levels.

Additionally, **Table 5** shows the *in vivo* anti-tumor efficacy. Administration of ADC-1 induced statistically significant (**p < 0.01) TGI (100% on day 35 post dosing) and TGD (>200% at the end of the study) compared to vehicle control at all dose levels tested. TGI reported in all instances is TGI "last" relating to the measure of "depth" of response. %TGI (tumor growth inhibition) = 1 - (mean tumor volume of treatment group/ mean tumor volume of treatment control group) x 100. For % TGI, P values (as indicated by asterisks) are derived from the test comparison of treatment group vs. treatment control group, following a log10 transformation of tumor volume data. The test for %TGI was performed on data from last day on which all animals in the control group are present/alive. TGD is a measure of response durability. % TGD (tumor growth delay) = (T - C)/ C x 100, where T is the median time to endpoint of treatment group and C is the median time to endpoint of treatment control group. For % TGD, P values (as indicated by asterisks) are derived from Kaplan Meier log-rank comparison of treatment group vs. treatment control group. Based on an endpoint of 1000 mm3. Fisher's Exact Test for Response Frequency was used for comparison of treatment group to that of treatment control group. CR: complete response, % of population with tumors < 25 mm3 for at least 3 consecutive measurements; PR: partial response, % of population with tumors > 25 mm3 and < 50% of the original tumor volume at onset of dosing for at least 3 consecutive measurements; OR: overall response = TFE + CR + PR; TFE: tumor free events, % of population with complete tumor regression at end of study as determined by palpation.

**Table 5: Growth Inhibition in DLBCL ABC CDX (U-2932) Tumors Following Treatment with ADC-1**

| **Compound(s)** | **Dose** | **TGI (%)** | **TGD (%)** | **CR (%)** | **PR (%)** | **TR (%)** |
|---|---|---|---|---|---|---|
| Vehicle | - | -- | | 0 | 0 | 0 |
| ADC-1 | 1x | 100** | >200** | 100 | 0 | 100 |
| ADC-1 | 2x | 100** | >200** | 100 | 0 | 100 |
| ADC-1 | 4x | 100** | >200** | 100 | 0 | 100 |

| | | | | | | |
|---|---|---|---|---|---|---|
| * p < 0.05; ** p <0.01; *** p < 0.001. | | | | | | |

### 2.6 Example 6: In Vivo Cytotoxicity

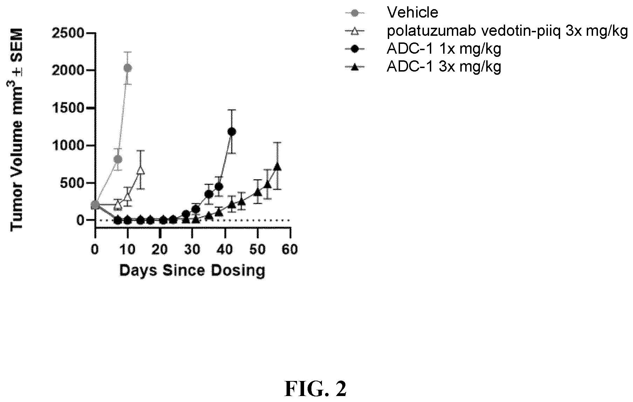

*In vivo* growth inhibition of DLBCL GCB CDX tumors (OCI Ly 19) treated with ADC-1 or polatuzumab vedotin-piiq was measured at a number of dose levels of ADC-1, including the initial dose level (1x) and three-times the initial dose level (3x), or polatuzumab vedotin-piiq, at 3x the initial dose level of ADC-1. Administration of each dose of ADC-1 or polatuzumab vedotin-piiq was as a single bolus intraperitoneally on day zero. As shown in **FIG. 2****,** ADC-1 administration to xenografted DLBCL GCB CDX tumors induced statistically significant tumor growth inhibition and tumor growth delay at all dose levels tested, compared to vehicle controls. CRs of 100% were observed at all dose levels. Polatuzumab vedotin-piiq administration to xenografted DLBCL GCB CDX tumors induced statistically significant tumor growth inhibition and tumor growth delay at the dose level tested, compared to vehicle controls. No CRs were observed at the dose level tested. ADC-1 administration to xenografted DLBCL GCB CDX tumors induced statistically significant tumor growth inhibition and tumor growth delay at all dose levels tested, compared to polatuzumab vedotin-piiq treated tumors.

Additionally, **Table 6** shows the *in vivo* anti-tumor efficacy. Administration of ADC-1 induced statistically significant (**p < 0.01) TGI (100% on day 10 post dosing) and TGD (>320% and >460% at the end of the study) at the 1x and 3x dose levels respectively, compared to vehicle controls. Administration of polatuzumab vedotin-piiq induced statistically significant (**p < 0.01) TGI (84% on day 10 post dosing) and TGD (90% at the end of the study) compared to vehicle controls. Administration of ADC-1 induced statistically significant (**p < 0.01) TGI (99% on day 14 post dosing) and TGD (>121% and >195% at the end of the study) at the 1x and 3x dose levels respectively, compared to polatuzumab vedotin-piiq.

**Table 6: Growth Inhibition in DLBCL GCB CDX (OCI Ly19) Tumors Following Treatment with ADC-1 or Polatuzumab vedotin-piiq**

| **Test Compound** | **Control Compound** | **Dose (Test Compound)** | **TGI (%)** | **TGD (%)** | **CR (%)** | **PR (%)** | **TR (%)** |
|---|---|---|---|---|---|---|---|
| Vehicle | Vehicle | - | - | | 0 | 0 | 0 |
| ADC-1 | Vehicle | 1x | 100** | >320** | 100 | 0 | 100 |
| ADC-1 | Vehicle | 3x | 100** | >460** | 100 | 0 | 100 |
| polatuzumab vedotin-piiq | Vehicle | 3x | 84** | 90** | 0 | 0 | 0 |
| ADC-1 | polatuzumab vedotin-piiq | 1x | 99** | >121** | 100 | 0 | 100 |
| ADC-1 | polatuzumab vedotin-piiq | 3x | 99** | >195** | 100 | 0 | 100 |

All patents and publications mentioned in this specification are incorporated herein by reference in their entireties. From the foregoing description, it will be apparent that variations and modifications can be made to the embodiments described herein to adopt it to various uses and conditions. Such embodiments are also within the scope of the following claims.

### SEQUENCE TABLE

| **SEQ ID NO** | **Description** | **Sequence** |
|---|---|---|
| 1 | Ab1 VH-CDR1 (Kabat) | NYWIT |
| 2 | Ab1 VH-CDR2 (Kabat) | NINQAGSEKSYLDAVKG |
| 3 | Ab1 VH-CDR3 (Kabat) | GFRAFDI |
| 4 | Ab1 VL-CDR1 (Kabat) | RSSQSLLHSSGESYLD |
| 5 | Ab1 VL-CDR2 (Kabat) | LGSHRAS |
| 6 | Ab1 VL-CDR3 (Kabat) | MQALQTPLT |
| 7 | Ab1 VH | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSS |
| 8 | Ab1 VL | DFVLTQSPLSLSVTPGEPASISCRSSQSLLHSSGESYLDWFLQR PGQSPQVLIYLGSHRASGAPDRFSGSGSGTDFTLKISRVEAED VGVYYCMQALQTPLTFGGGTRVEIK |
| 9 | Ab1 heavy chain | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDTLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVMHEALHNHYTQKSLSLSPGK |
| 10 | Ab1 light chain | DFVLTQSPLSLSVTPGEPASISCRSSQSLLHSSGESYLDWFLQR PGQSPQVLIYLGSHRASGAPDRFSGSGSGTDFTLKISRVEAED VGVYYCMQALQTPLTFGGGTRVEIKRTVAAPSVFIFPPSDEQL KSGTASVVCLLNNFYPREAKVQWKVDNALQSGNSQESVTEQ |
| | | DSKDSTYSLSSTLTLSKADYEKHKVYACEVTHQGLSSPVTKSF NRGEA |
| 11 | polynucleotide encoding Ab1 heavy chain | ATGGAGTTCGGACTCTCCTGGCTGTTTCTGGTTGCGAT CCTCAAGGGCGTGCAGTGCgaagtgcagttggtggagtccggtggtggtc tggtccagcctggcggatctctgcgcctgccatgtgccgccagcgggttcaccttctacaact actggattacctggattagacaggcgccaggaaagggcctcgaatgggtggccaatatcaa ccaggccggatccgaaaagagctacctcgatgctgtcaaggggcggttcaccatctcgcgg gataacgccaagaattccgtgtacctccaaatgaacagcctgagggtggaggacaccgca gtgtattactgcgccggaggtttccgcgcattcgatatctggggccaggggactatggtcacc gtcagctcggcctccacaaagggaccatcggtgtttccgttggcccctcgtcaaagtcgacct caggaggcaccgctgctctggggtgcctggtcaaggattacttccccgaaccagtgaccgtgt cctggaactccggcgcgctgactagcggagtgcatactttccccgcggtgcttcaaagctccgg cttgtactctctgtcctccgtcgtgaccgtgccctcctcatcgctgggaacccagacctacatctg caacgtgaaccataagcccagcaacaccaaggtcgacaagaaggtcgagccgaagtcctg cgacaagactcacacttgcccgccttgtcctgccccggagcttctgggaggaccgtccgtgttc ctgttcccgccgaagcccaaggacaccctgatgatttcccgcacccccgaagtcacttgcgtgg tggtggacgtgtcccacgaggatcctgaggtcaagttcaattggtacgtggacggcgtggagg tgcacaacgccaagactaagccccgcgaggaacagtacaactccacctaccgggtcgtcag cgtgctgactgtgctgcaccaagactggctcaacggaaaggagtacaagtgcaaagtgtcca acaaggcactgcctgcccctatcgaaaagaccatcagcaaggccaaaggacagcctaggg agccccaagtgtatacgctgccaccttcgcgggaagagatgacgaagaaccaagtgtcgctg acttgccttgtgaagggcttctacccgtccgacattgcggtggaatgggaatccaacgggcagc ccgaaaacaactacaaaaccaccccgccggtactcgactcggacgggtcctttttcctgtactc aaagctcaccgtggacaagtccagatggcagcagggcaacgtgttctcctgttccgtgatgcat gaagccctgcacaaccactacactcagaagtcactgagcctgtcaccgggaaaatga |
| 12 | polynucleotide encoding Ab1 light chain | ATGGACATGCGCGTGCCAGCACAGCTGCTCGGGCTGCT GCTCCTCTGGTTCCCGGGAAGCCGCTGTgactttgtgctgaccca gtcgcccctgagcttgtccgtgactccgggagaacccgcctcaattagctgccggtcatcgc aatccctgctgcattcctcgggagagtcgtacctggactggttcttgcaacggcccggtcaat cccctcaagtgcttatctacctcggatcgcacagagccagcggagcacctgatcggttctctg ggtcgggatcaggcaccgacttcacactgaaaatctcccgggtggaggctgaggacgtcgg ggtgtactactgcatgcaggcgctgcagactcccctgaccttcggtggaggcaccagggtg gagattaagagaaccgtggctgcccctagcgtgttcatctttccgccatccgatgagcagctga agtccggtaccgcctccgtcgtgtgcctgcttaacaacttctacccgcgcgaagccaaggtcca gtggaaggtcgataacgcgctgcagtccggaaactcacaggaaagcgtgaccgaacagga ctccaaggactccacctactcgctctcctccactctcacgctgtccaaggcggactacgagaag cacaaggtctatgcctgcgaagtgacccaccagggcctgagctcccctgtgactaagagcttc aataggggcgaagcctga |
| 13 | Ab1 heavy chain without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDTLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA |
| | | VEWESNGQPENNYKTTPPVLDSDGSFFLYSKLTVDKSRWQQ GNVFSCSVMHEALHNHYTQKSLSLSPG |
| 14 | Ab1 VH-CDR1 (IMGT) | GFTFYNYW |
| 15 | Ab1 VH-CDR2 (IMGT) | INQAGSEK |
| 16 | Ab1 VH-CDR3 (IMGT) | AGGFRAFDI |
| 17 | Ab1 VL-CDR1 (IMGT) | QSLLHSSGESY |
| 18 | Ab1 VL-CDR2 (IMGT) | LGS |
| 19 | Ab1 VL-CDR3 (IMGT) | MQALQTPLT |
| 20 | Ab1 heavy chain IgG1 STR | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDTLMIS RTPEVTCVVVDVSHEDPEVKFNWYVDGVEVHNAKTKPREEQ YNSTYRVVSVLTVLHQDWLNGKEYKCKVSNKALPAPIEKTIS KAKGQPREPQVYTLPPSREEMTKNQVSLTCLVKGFYPSDIAV EWESNGQPENNYKTTPPVLDSDGSFFLYSKLTVDKSRWQQG NVFSCSVMHEALHNHYTQKSLSLSPGK |
| 21 | Ab1 heavy chain IgG1 STR without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDTLMIS RTPEVTCVVVDVSHEDPEVKFNWYVDGVEVHNAKTKPREEQ YNSTYRVVSVLTVLHQDWLNGKEYKCKVSNKALPAPIEKTIS KAKGQPREPQVYTLPPSREEMTKNQVSLTCLVKGFYPSDIAV EWESNGQPENNYKTTPPVLDSDGSFFLYSKLTVDKSRWQQG NVFSCSVMHEALHNHYTQKSLSLSPG |
| 22 | Ab1 heavy chain IgG1 LALA | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDTLMI |
| | | SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVMHEALHNHYTQKSLSLSPGK |
| 23 | Ab1 heavy chain IgG1 LALA without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDTLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVMHEALHNHYTQKSLSLSPG |
| 24 | Ab1 heavy chain IgG1 YTE | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDTLYIT REPEVTCVVVDVSHEDPEVKFNWYVDGVEVHNAKTKPREEQ YNSTYRVVSVLTVLHQDWLNGKEYKCKVSNKALPAPIEKTIS KAKGQPREPQVYTLPPSREEMTKNQVSLTCLVKGFYPSDIAV EWESNGQPENNYKTTPPVLDSDGSFFLYSKLTVDKSRWQQG NVFSCSVMHEALHNHYTQKSLSLSPGK |
| 25 | Ab1 heavy chain IgG1 YTE without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDTLYIT REPEVTCVVVDVSHEDPEVKFNWYVDGVEVHNAKTKPREEQ YNSTYRVVSVLTVLHQDWLNGKEYKCKVSNKALPAPIEKTIS KAKGQPREPQVYTLPPSREEMTKNQVSLTCLVKGFYPSDIAV EWESNGQPENNYKTTPPVLDSDGSFFLYSKLTVDKSRWQQG NVFSCSVMHEALHNHYTQKSLSLSPG |
| 26 | Ab1 heavy chain IgG1 STR and YTE | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDTLYIT REPEVTCVVVDVSHEDPEVKFNWYVDGVEVHNAKTKPREEQ YNSTYRVVSVLTVLHQDWLNGKEYKCKVSNKALPAPIEKTIS |
| | | KAKGQPREPQVYTLPPSREEMTKNQVSLTCLVKGFYPSDIAV EWESNGQPENNYKTTPPVLDSDGSFFLYSKLTVDKSRWQQG NVFSCSVMHEALHNHYTQKSLSLSPGK |
| 27 | Ab1 heavy chain IgG1 STR and YTE without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDTLYIT REPEVTCVVVDVSHEDPEVKFNWYVDGVEVHNAKTKPREEQ YNSTYRVVSVLTVLHQDWLNGKEYKCKVSNKALPAPIEKTIS KAKGQPREPQVYTLPPSREEMTKNQVSLTCLVKGFYPSDIAV EWESNGQPENNYKTTPPVLDSDGSFFLYSKLTVDKSRWQQG NVFSCSVMHEALHNHYTQKSLSLSPG |
| 28 | Ab1 heavy chain IgG1 LALA and YTE | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDTLYI TREPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVMHEALHNHYTQKSLSLSPGK |
| 29 | Ab1 heavy chain IgG1 LALA and YTE without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDTLYI TREPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVMHEALHNHYTQKSLSLSPG |
| 30 | Ab1 heavy chain IgG1 REW | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDTLMI SRTPEVTCVVVDVSHEDPEVKFNWYVDGVEVHNAKTKPREE QYNSTYRVVSVLTVLHRDWLNGKEYKCKVSNKALPAPIEKTI SKAKGQPREPQVYTLPPSREEMTKNQVSLTCLVKGFYPSDIA |
| | | VEWESNGQPENNYKTTPPVLDSDGSFFLYSKLTVDKSRWQQ GNVFSCSVEHEALHWHYTQKSLSLSPGK |
| 31 | Ab1 heavy chain IgG1 REW without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDTLMI SRTPEVTCVVVDVSHEDPEVKFNWYVDGVEVHNAKTKPREE QYNSTYRVVSVLTVLHRDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVEHEALHWHYTQKSLSLSPG |
| 32 | Ab1 heavy chain IgG1 STR and REW | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDTLMIS RTPEVTCVVVDVSHEDPEVKFNWYVDGVEVHNAKTKPREEQ YNSTYRVVSVLTVLHRDWLNGKEYKCKVSNKALPAPIEKTIS KAKGQPREPQVYTLPPSREEMTKNQVSLTCLVKGFYPSDIAV EWESNGQPENNYKTTPPVLDSDGSFFLYSKLTVDKSRWQQG NVFSCSVEHEALHWHYTQKSLSLSPGK |
| 33 | Ab1 heavy chain IgG1 STR and REW without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDTLMIS RTPEVTCVVVDVSHEDPEVKFNWYVDGVEVHNAKTKPREEQ YNSTYRVVSVLTVLHRDWLNGKEYKCKVSNKALPAPIEKTIS KAKGQPREPQVYTLPPSREEMTKNQVSLTCLVKGFYPSDIAV EWESNGQPENNYKTTPPVLDSDGSFFLYSKLTVDKSRWQQG NVFSCSVEHEALHWHYTQKSLSLSPG |
| 34 | Ab1 heavy chain IgG1 LALA and REW | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDTLMI SRTPEVTCVVVDVSHEDPEVKFNWYVDGVEVHNAKTKPREE QYNSTYRVVSVLTVLHRDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVEHEALHWHYTQKSLSLSPGK |
| 35 | Ab1 heavy chain IgG1 LALA and REW without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDTLMI SRTPEVTCVVVDVSHEDPEVKFNWYVDGVEVHNAKTKPREE QYNSTYRVVSVLTVLHRDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVEHEALHWHYTQKSLSLSPG |
| 36 | Ab1 heavy chain IgG1 LS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDTLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVLHEALHSHYTQKSLSLSPGK |
| 37 | Ab1 heavy chain IgG1 LS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDTLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVLHEALHSHYTQKSLSLSPG |
| 38 | Ab1 heavy chain IgG1 STR and LS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDTLMIS RTPEVTCVVVDVSHEDPEVKFNWYVDGVEVHNAKTKPREEQ YNSTYRVVSVLTVLHQDWLNGKEYKCKVSNKALPAPIEKTIS KAKGQPREPQVYTLPPSREEMTKNQVSLTCLVKGFYPSDIAV EWESNGQPENNYKTTPPVLDSDGSFFLYSKLTVDKSRWQQG NVFSCSVLHEALHSHYTQKSLSLSPGK |
| 39 | Ab1 heavy chain IgG1 | EVQLVESGGGLVQPGGSLRLPCAASGFTFYNYWITWIRQAPG KGLEWVANINQAGSEKSYLDAVKGRFTISRDNAKNSVYLQM |
| | STR and LS without terminal lysine | NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDTLMIS RTPEVTCVVVDVSHEDPEVKFNWYVDGVEVHNAKTKPREEQ YNSTYRVVSVLTVLHQDWLNGKEYKCKVSNKALPAPIEKTIS KAKGQPREPQVYTLPPSREEMTKNQVSLTCLVKGFYPSDIAV EWESNGQPENNYKTTPPVLDSDGSFFLYSKLTVDKSRWQQG NVFSCSVLHEALHSHYTQKSLSLSPG |
| 40 | Ab1 heavy chain IgG1 LALA and LS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDTLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVLHEALHSHYTQKSLSLSPGK |
| 41 | Ab1 heavy chain IgG1 LALA and LS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDTLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVLHEALHSHYTQKSLSLSPG |
| 42 | Ab1 heavy chain IgG1 QL | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDQLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVLHEALHNHYTQKSLSLSPGK |
| 43 | Ab1 heavy chain IgG1 QL without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV |
| | | HTFPAVLQSSGLYSLSSVVTVPSSSLGTQTYICNVNHKPSNTK VDKKVEPKSCDKTHTCPPCPAPELLGGPSVFLFPPKPKDQLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVLHEALHNHYTQKSLSLSPG |
| 44 | Ab1 heavy chain IgG1 STR and QL | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDQLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVLHEALHNHYTQKSLSLSPGK |
| 45 | Ab1 heavy chain IgG1 STR and QL without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDQLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVLHEALHNHYTQKSLSLSPG |
| 46 | Ab1 heavy chain IgG1 LALA and QL | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDQLMI SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVLHEALHNHYTQKSLSLSPGK |
| 47 | Ab1 heavy chain IgG1 LALA and QL without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDQLMI |
| | | SRTPEVTCVVVDVSHEDPEVKFNWYVDGVEVHNAKTKPREE QYNSTYRVVSVLTVLHQDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVLHEALHNHYTQKSLSLSPG |
| 48 | Ab1 heavy chain IgG1 DHS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDTLMI SRTPEVTCVVVDVSHEDPEVKFNWYVDGVEVHNAKTKPREE QYNSTYRVVSVLTVDHHDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVMHEALHSHYTQKSLSLSPGK |
| 49 | Ab1 heavy chain IgG1 DHS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPELLGGPSVFLFPPKPKDTLMI SRTPEVTCVVVDVSHEDPEVKFNWYVDGVEVHNAKTKPREE QYNSTYRVVSVLTVDHHDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVMHEALHSHYTQKSLSLSPG |
| 50 | Ab1 heavy chain IgG1 STR and DHS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDTLMIS RTPEVTCVVVDVSHEDPEVKFNWYVDGVEVHNAKTKPREEQ YNSTYRVVSVLTVDHHDWLNGKEYKCKVSNKALPAPIEKTIS KAKGQPREPQVYTLPPSREEMTKNQVSLTCLVKGFYPSDIAV EWESNGQPENNYKTTPPVLDSDGSFFLYSKLTVDKSRWQQG NVFSCSVMHEALHSHYTQKSLSLSPGK |
| 51 | Ab1 heavy chain IgG1 STR and DHS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPESTRGPSVFLFPPKPKDTLMIS RTPEVTCVVVDVSHEDPEVKFNWYVDGVEVHNAKTKPREEQ YNSTYRVVSVLTVDHHDWLNGKEYKCKVSNKALPAPIEKTIS |
| | | KAKGQPREPQVYTLPPSREEMTKNQVSLTCLVKGFYPSDIAV EWESNGQPENNYKTTPPVLDSDGSFFLYSKLTVDKSRWQQG NVFSCSVMHEALHSHYTQKSLSLSPG |
| 52 | Ab1 heavy chain IgG1 LALA and DHS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDTLMI SRTPEVTCVVVDVSHEDPEVKFNWYVDGVEVHNAKTKPREE QYNSTYRVVSVLTVDHHDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVMHEALHSHYTQKSLSLSPGK |
| 53 | Ab1 heavy chain IgG1 LALA and DHS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPSSKSTSGGTAALGCLVKDYFPEPVTVSWNSGALTSGV HTFPAVLQSSGLYSLSSVVTVPSSSLGTQTYICNVNHKPSNTK VDKKVEPKSCDKTHTCPPCPAPEAAGGPSVFLFPPKPKDTLMI SRTPEVTCVVVDVSHEDPEVKFNWYVDGVEVHNAKTKPREE QYNSTYRVVSVLTVDHHDWLNGKEYKCKVSNKALPAPIEKTI SKAKGQPREPQVYTLPPSREEMTKNQVSLTCLVKGFYPSDIA VEWESNGQPENNYKTTPPVLDSDGSFFLYSKLTVDKSRWQQ GNVFSCSVMHEALHSHYTQKSLSLSPG |
| 54 | Ab1 heavy chain IgG4 STR | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 55 | Ab1 heavy chain IgG4 STR without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES |
| | | NGQPENNYKTTPPVLDSDGSFFLYSRLTVDKSRWQEGNVFSC SVMHEALHNHYTQKSLSLSLG |
| 56 | Ab1 heavy chain IgG4 FALA | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 57 | Ab1 heavy chain IgG4 FALA without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |
| 58 | Ab1 heavy chain IgG4 YTE | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 59 | Ab1 heavy chain IgG4 YTE without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |
| 60 | Ab1 heavy chain IgG4 STR and YTE | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 61 | Ab1 heavy chain IgG4 STR and YTE without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |
| 62 | Ab1 heavy chain IgG4 FALA and YTE | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 63 | Ab1 heavy chain IgG4 FALA and YTE without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |
| 64 | Ab1 heavy chain IgG4 REW | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLGK |
| 65 | Ab1 heavy chain IgG4 REW without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLG |
| 66 | Ab1 heavy chain IgG4 STR and REW | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLGK |
| 67 | Ab1 heavy chain IgG4 STR and REW without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLG |
| 68 | Ab1 heavy chain IgG4 | EVQLVESGGGLVQPGGSLRLPCAASGFTFYNYWITWIRQAPG KGLEWVANINQAGSEKSYLDAVKGRFTISRDNAKNSVYLQM |
| | FALA and REW | NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLGK |
| 69 | Ab1 heavy chain IgG4 FALA and REW without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLG |
| 70 | Ab1 heavy chain IgG4 LS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLGK |
| 71 | Ab1 heavy chain IgG4 LS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLG |
| 72 | Ab1 heavy chain IgG4 STR and LS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH |
| | | TFPAVLQSSGLYSLSSVVTVPSSSLGTKTYTCNVDHKPSNTKV DKRVESKYGPPCPSCPAPESTRGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLGK |
| 73 | Ab1 heavy chain IgG4 STR and LS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLG |
| 74 | Ab1 heavy chain IgG4 FALA and LS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLGK |
| 75 | Ab1 heavy chain IgG4 FALA and LS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLG |
| 76 | Ab1 heavy chain IgG4 QL | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDQLMISRTPE |
| | | VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHNHYTQKSLSLSLGK |
| 77 | Ab1 heavy chain IgG4 QL without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDQLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHNHYTQKSLSLSLG |
| 78 | Ab1 heavy chain IgG4 STR and QL | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDQLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHNHYTQKSLSLSLGK |
| 79 | Ab1 heavy chain IgG4 STR and QL without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDQLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHNHYTQKSLSLSLG |
| 80 | Ab1 heavy chain IgG4 FALA and QL | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDQLMISRTP EVTCVVVDVSQEDPEVQFNWYVDGVEVHNAKTKPREEQFNS TYRVVSVLTVLHQDWLNGKEYKCKVSNKGLPSSIEKTISKAK |
| | | GQPREPQVYTLPPSQEEMTKNQVSLTCLVKGFYPSDIAVEWE SNGQPENNYKTTPPVLDSDGSFFLYSRLTVDKSRWQEGNVFS CSVLHEALHNHYTQKSLSLSLGK |
| 81 | Ab1 heavy chain IgG4 FALA and QL without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDQLMISRTP EVTCVVVDVSQEDPEVQFNWYVDGVEVHNAKTKPREEQFNS TYRVVSVLTVLHQDWLNGKEYKCKVSNKGLPSSIEKTISKAK GQPREPQVYTLPPSQEEMTKNQVSLTCLVKGFYPSDIAVEWE SNGQPENNYKTTPPVLDSDGSFFLYSRLTVDKSRWQEGNVFS CSVLHEALHNHYTQKSLSLSLG |
| 82 | Ab1 heavy chain IgG4 DHS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLGK |
| 83 | Ab1 heavy chain IgG4 DHS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLG |
| 84 | Ab1 heavy chain IgG4 STR and DHS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES |
| | | NGQPENNYKTTPPVLDSDGSFFLYSRLTVDKSRWQEGNVFSC SVMHEALHSHYTQKSLSLSLGK |
| 85 | Ab1 heavy chain IgG4 STR and DHS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPESTRGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLG |
| 86 | Ab1 heavy chain IgG4 FALA and DHS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLGK |
| 87 | Ab1 heavy chain IgG4 FALA and DHS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEAAGGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLG |
| 88 | Ab1 heavy chain IgG4 S228P and STR | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 89 | Ab1 heavy chain IgG4 S228P and STR without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |
| 90 | Ab1 heavy chain IgG4 S228P and FALA | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 91 | Ab1 heavy chain IgG4 S228P and FALA without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |
| 92 | Ab1 heavy chain IgG4 S228P and YTE | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 93 | Ab1 heavy chain IgG4 | EVQLVESGGGLVQPGGSLRLPCAASGFTFYNYWITWIRQAPG KGLEWVANINQAGSEKSYLDAVKGRFTISRDNAKNSVYLQM |
| | S228P and YTE without terminal lysine | NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |
| 94 | Ab1 heavy chain IgG4 S228P and STR and YTE | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 95 | Ab1 heavy chain IgG4 S228P and STR and YTE without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |
| 96 | Ab1 heavy chain IgG4 S228P and FALA and YTE | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 97 | Ab1 heavy chain IgG4 S228P and FALA and | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH |
| | YTE without terminal lysine | TFPAVLQSSGLYSLSSVVTVPSSSLGTKTYTCNVDHKPSNTKV DKRVESKYGPPCPPCPAPEAAGGPSVFLFPPKPKDTLYITREPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |
| 98 | Ab1 heavy chain IgG4 S228P and REW | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLGK |
| 99 | Ab1 heavy chain IgG4 S228P and REW without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLG |
| 100 | Ab1 heavy chain IgG4 S228P and STR and REW | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLGK |
| 101 | Ab1 heavy chain IgG4 S228P and STR and REW without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDTLMISRTPE |
| | | VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLG |
| 102 | Ab1 heavy chain IgG4 S228P and FALA and REW | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLGK |
| 103 | Ab1 heavy chain IgG4 S228P and FALA and REW without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDTLMISRTPE VTCVVVDVSQEDPEVQFNWYVDGVEVHNAKTKPREEQFNST YRVVSVLTVLHRDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVEHEALHWHYTQKSLSLSLG |
| 104 | Ab1 heavy chain IgG4 S228P and LS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLGK |
| 105 | Ab1 heavy chain IgG4 S228P and LS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG |
| | | QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLG |
| 106 | Ab1 heavy chain IgG4 S228P and STR and LS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLGK |
| 107 | Ab1 heavy chain IgG4 S228P and STR and LS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLG |
| 108 | Ab1 heavy chain IgG4 S228P and FALA and LS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHSHYTQKSLSLSLGK |
| 109 | Ab1 heavy chain IgG4 S228P and FALA and LS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES |
| | | NGQPENNYKTTPPVLDSDGSFFLYSRLTVDKSRWQEGNVFSC SVLHEALHSHYTQKSLSLSLG |
| 110 | Ab1 heavy chain IgG4 S228P and QL | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDQLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHNHYTQKSLSLSLGK |
| 111 | Ab1 heavy chain IgG4 S228P and QL without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDQLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHNHYTQKSLSLSLG |
| 112 | Ab1 heavy chain IgG4 S228P and STR and QL | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDQLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHNHYTQKSLSLSLGK |
| 113 | Ab1 heavy chain IgG4 S228P and STR and QL without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDQLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVLHEALHNHYTQKSLSLSLG |
| 114 | Ab1 heavy chain IgG4 S228P and FALA and QL | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDQLMISRTP EVTCVVVDVSQEDPEVQFNWYVDGVEVHNAKTKPREEQFNS TYRVVSVLTVLHQDWLNGKEYKCKVSNKGLPSSIEKTISKAK GQPREPQVYTLPPSQEEMTKNQVSLTCLVKGFYPSDIAVEWE SNGQPENNYKTTPPVLDSDGSFFLYSRLTVDKSRWQEGNVFS CSVLHEALHNHYTQKSLSLSLGK |
| 115 | Ab1 heavy chain IgG4 S228P and FALA and QL without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDQLMISRTP EVTCVVVDVSQEDPEVQFNWYVDGVEVHNAKTKPREEQFNS TYRVVSVLTVLHQDWLNGKEYKCKVSNKGLPSSIEKTISKAK GQPREPQVYTLPPSQEEMTKNQVSLTCLVKGFYPSDIAVEWE SNGQPENNYKTTPPVLDSDGSFFLYSRLTVDKSRWQEGNVFS CSVLHEALHNHYTQKSLSLSLG |
| 116 | Ab1 heavy chain IgG4 S228P and DHS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLGK |
| 117 | Ab1 heavy chain IgG4 S228P and DHS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLG |
| 118 | Ab1 heavy chain IgG4 | EVQLVESGGGLVQPGGSLRLPCAASGFTFYNYWITWIRQAPG KGLEWVANINQAGSEKSYLDAVKGRFTISRDNAKNSVYLQM |
| | S228P and STR and DHS | NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLGK |
| 119 | Ab1 heavy chain IgG4 S228P and STR and DHS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPESTRGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLG |
| 120 | Ab1 heavy chain IgG4 S228P and FALA and DHS | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLGK |
| 121 | Ab1 heavy chain IgG4 S228P and FALA and DHS without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEAAGGPSVFLFPPKPKDTLMISRTPE VTCVVVDVSQEDPEVQFNWYVDGVEVHNAKTKPREEQFNST YRVVSVLTVDHHDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHSHYTQKSLSLSLG |
| 122 | Ab1 heavy chain IgG4 | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH |
| | | TFPAVLQSSGLYSLSSVVTVPSSSLGTKTYTCNVDHKPSNTKV DKRVESKYGPPCPSCPAPEFLGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 123 | Ab1 heavy chain IgG4 without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPSCPAPEFLGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |
| 124 | Ab1 heavy chain IgG4 228P | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLGK |
| 125 | Ab1 heavy chain IgG4 228P without terminal lysine | EVQLVESGGGLVQPGGSLRLPCAASGFTFYNYWITWIRQAPG KGLEWVANINQAGSEKSYLDAVKGRFTISRDNAKNSVYLQM NSLRVEDTAVYYCAGGFRAFDIWGQGTMVTVSSASTKGPSV FPLAPCSRSTSESTAALGCLVKDYFPEPVTVSWNSGALTSGVH TFPAVLQSSGLYSLSSVVTVPSSSLGTKTYTCNVDHKPSNTKV DKRVESKYGPPCPPCPAPEFLGGPSVFLFPPKPKDTLMISRTPE VTCVVVDVSQEDPEVQFNWYVDGVEVHNAKTKPREEQFNST YRVVSVLTVLHQDWLNGKEYKCKVSNKGLPSSIEKTISKAKG QPREPQVYTLPPSQEEMTKNQVSLTCLVKGFYPSDIAVEWES NGQPENNYKTTPPVLDSDGSFFLYSRLTVDKSRWQEGNVFSC SVMHEALHNHYTQKSLSLSLG |

## Claims

1. An anti-CD79b antibody-drug conjugate (ADC) of the following structure: 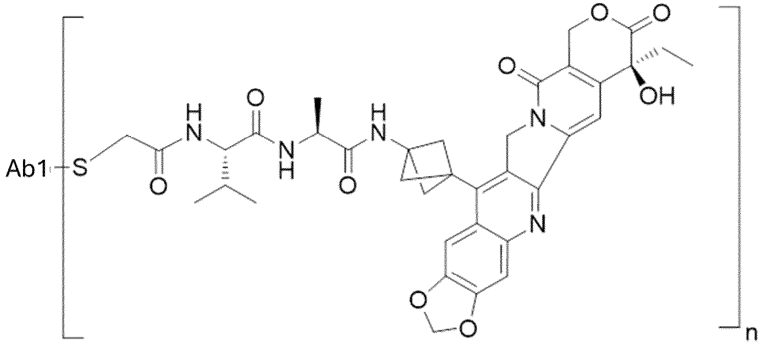
wherein n is an integer from 1 to 8,
wherein Ab1 is an anti-CD79b antibody comprising a heavy chain variable region comprising a VH-CDR1, a VH-CDR2, and a VH-CDR3; and a light chain variable region comprising a VL-CDR1, a VL-CDR2, and a VL-CDR3; and
wherein:
VH-CDR1 has the amino acid sequence of SEQ ID NO: 1;
VH-CDR2 has the amino acid sequence of SEQ ID NO: 2;
VH-CDR3 has the amino acid sequence of SEQ ID NO: 3;
VL-CDR1 has the amino acid sequence of SEQ ID NO: 4;
VL-CDR2 has the amino acid sequence of SEQ ID NO: 5; and
VL-CDR3 has the amino acid sequence of SEQ ID NO: 6.

2. The anti-CD79b ADC of claim 1, wherein Ab1 is an IgG₁ isotype and comprises: a heavy chain variable region having the amino acid sequence of SEQ ID NO: 7; and a light chain variable region having the amino acid sequence of SEQ ID NO: 8.

3. The anti-CD79b ADC of claim 1, wherein Ab1 is an IgG₁ isotype and comprises: a heavy chain having the amino acid sequence of SEQ ID NO: 9 or SEQ ID NO: 13; and a light chain having the amino acid sequence of SEQ ID NO: 10.

4. The anti-CD79b ADC of claim 3, wherein Ab1 comprises: a heavy chain having the amino acid sequence of SEQ ID NO: 13; and a light chain having the amino acid sequence of SEQ ID NO: 10.

5. The anti-CD79b ADC of claim 4, wherein n is 6.

6. The anti-CD79b ADC of claim 3, wherein Ab1 comprises: a heavy chain having the amino acid sequence of SEQ ID NO: 9; and a light chain having the amino acid sequence of SEQ ID NO: 10.

7. The anti-CD79b ADC of claim 6, wherein n is 6.

8. The anti-CD79b ADC of claim 1 or 2, wherein the anti-CD79b antibody comprises two identical heavy chains and two identical light chains, wherein each heavy chain comprises the amino acid sequence of SEQ ID NO: 9 or SEQ ID NO: 13; and wherein each light chain comprises the amino acid sequence of SEQ ID NO: 10.

9. The anti-CD79b ADC of claim 1 or 2, wherein the anti-CD79b antibody comprises a human kappa light chain constant region.

10. A pharmaceutical composition comprising the anti-CD79b ADC of any one of claims 1-9 and a pharmaceutically acceptable carrier.

11. A method of treating a disease, the method comprising administering an anti-CD79b ADC of claims 1-9 to a patient in need thereof.

12. An antibody that specifically binds to human CD79b, wherein the antibody comprises: a heavy chain variable region comprising the amino acid sequence of SEQ ID NO:7; and a light chain variable region comprising the amino acid sequence of SEQ ID NO:8.

13. The antibody of claim 12, wherein the antibody is an IgG₁ isotype and the VH-CDR1 comprises the amino acid sequence of SEQ ID NO: 1, the VH-CDR2 comprises the amino acid sequence of SEQ ID NO: 2, the VH-CDR3 comprises the amino acid sequence of SEQ ID NO: 3, the VL-CDR1 comprises the amino acid sequence of SEQ ID NO: 4, the VL-CDR2 comprises the amino acid sequence of SEQ ID NO: 5, and the VL-CDR3 comprises the amino acid sequence of SEQ ID NO: 6.

14. The antibody of claim 12 or 13, wherein the antibody comprises a human kappa light chain constant region.

15. The antibody of any one of claims 12-14, wherein the antibody comprises a heavy chain variable region comprising the amino acid sequence of SEQ ID NO: 7 and a light chain variable region comprising the amino acid sequence of SEQ ID NO: 8.

16. The antibody of any one of claims 12-15, wherein the antibody comprises a heavy chain comprising the amino acid sequence of SEQ ID NO: 9 or SEQ ID NO: 13 and a light chain comprising the amino acid sequence of SEQ ID NO: 10.

17. The antibody of claim 16, wherein the antibody comprises a heavy chain comprising the amino acid sequence of SEQ ID NO: 13 and a light chain comprising the amino acid sequence of SEQ ID NO: 10.

18. The antibody of claim 16, wherein the antibody comprises a heavy chain comprising the amino acid sequence of SEQ ID NO: 9 and a light chain comprising the amino acid sequence of SEQ ID NO: 10.

19. A polynucleotide or a set of polynucleotides encoding the antibody of any one of claims 12-18 or a portion thereof.

20. A vector or a set of vectors comprising the polynucleotide or set of polynucleotides of claim 19.

21. A host cell comprising the polynucleotide or set of polynucleotides of claim 19 or the vector or set of vectors of claim 20.

22. A pharmaceutical composition comprising the antibody of any one of claims 12-18 and a pharmaceutically acceptable carrier.

23. A process for producing an anti-CD79b antibody-drug conjugate, comprising conjugating an anti-CD79b antibody of any one of claims 12-18 to a Topli payload shown as Formula 2 via a linker shown as Formula 3.

24. A composition comprising an anti-CD79b antibody-drug conjugate wherein the DAR is about 4.
